# FASCICULE DE BREVET EUROPEEN

(11) **EP 3 999 520 B1**
(45) Date de publication et mention de la délivrance du brevet: **30.08.2023**
(21) Numéro de dépôt: 20742690.9
(22) Date de dépôt: 15.07.2020
(51) Int. Cl.: C07H 21/00, C12N 15/11, A61P 1/00

(54) **COMPLEXE HYBRIDE HTIARN / NANOPARTICULE ET SON UTILISATION POUR TRAITEMENT D'UNE MALADIE DU SYSTÈME DIGESTIF**
HYBRIDER HTIRNA-/NANOPARTIKEL-KOMPLEX UND SEINE VERWENDUNG ZUR BEHANDLUNG EINER KRANKHEIT DES VERDAUUNGSSYSTEMS
HYBRID HTIRNA/NANOPARTICLE COMPLEX AND USE THEREOF FOR TREATING A DISEASE OF THE DIGESTIVE SYSTEM

(30) Priorité: 15.07.2019 FR 1907965
(43) Date de publication de la demande: 25.05.2022
(73) Titulaire: COMMISSARIAT À L'ÉNERGIE ATOMIQUE ET AUX ÉNERGIES ALTERNATIVES, 75015 Paris (FR)
(72) Inventeur: NAVARRO Y GARCIA, Fabrice, 38054 Grenoble cedex 09 (FR); JARY, Dorothée, 38054 Grenoble cedex 09 (FR); NOUGAREDE, Adrien, 38054 Grenoble cedex 09 (FR)
(74) Mandataire: Lavoix
(86) Numéro de dépôt international: PCT/EP2020/070022
(87) Numéro de publication internationale: WO 2021/009242

(56) Documents cités:
- FR-A1- 2 994 849
- US-A1- 2016 208 245
- KIRILL A. AFONIN ET AL: "Triggering of RNA Interference with RNA-RNA, RNA-DNA, and DNA-RNA Nanoparticles", ACS NANO, 18 décembre 2014 (2014-12-18), XP055164747, ISSN: 1936-0851, DOI: 10.1021/nn504508s
- KRISTEN L. KOZIELSKI ET AL: "Bioengineered nanoparticles for siRNA delivery", WILEY INTERDISCIPLINARY REVIEWS: NANOMEDICINE AND NANOBIOTECHNOLOGY, 1 juillet 2013 (2013-07-01), pages n/a-n/a, XP055160902, ISSN: 1939-5116, DOI: 10.1002/wnan.1233

## Description

La présente invention concerne un complexe de molécules hybrides ADN/ARN, appelée « hybrides htiARN », et de nanoparticules et son utilisation thérapeutique, notamment pour traitement d'une maladie du système digestif.

Les siARN sont de plus en plus utilisées comme candidats médicaments. Les siARN comprennent deux brins d'ARN généralement d'une longueur de 21 à 23 nucléotides chacun, dont :
- un brin antisens, qui peut s'apparier par complémentarité avec l'ARN messager d'un gène cible, et
- un brin sens ou passager, complémentaire au brin antisens.
En se fixant sur l'ARN messager d'un gène cible, le brin antisens d'un siARN va provoquer l'inhibition fonctionnelle de l'expression de ce gène cible via le mécanisme d'ARN interférence, le brin sens n'étant pas utilisé au cours de ce processus. Ce mécanisme d'ARN interférence peut ainsi servir à moduler l'expression du gène cible dans un but thérapeutique.

La barrière la plus importante à l'utilisation des siARN comme médicament est leur très faible biodisponibilité. Pour améliorer les propriétés pharmacocinétiques et assurer la biodisponibilité intracellulaire d'une molécule active hydrophile telle qu'un siARN, il est possible de l'associer à une nanoparticule lipidique (LNP), qui va protéger le siARN, permettre son transport et ainsi d'atteindre sa cible. En particulier, la fixation d'un siARN, qui est une molécule anionique (charge nette négative), est possible sur une nanoparticule lipidique cationique (charge nette positive) par interaction électrostatique.

Les nanoparticules lipidiques peuvent être sous forme isolée ou être les gouttelettes d'une émulsion dans laquelle la phase dispersée est lipidique.

La demande FR 2 994 849 décrit une nanoémulsion, comprenant une phase aqueuse continue et une phase dispersée sous forme de gouttelettes comprenant notamment un tensioactif cationique. Ce tensioactif cationique permet la complexation de siARN sur les LNP de la phase dispersée.

La demande US2016/0208245 décrit des hybrides ARN/ADN sous forme de nanoparticules pour le transport de siARN.

Afonin et al.

L'efficacité du transport d'un siARN par une nanoparticule dépend principalement de la stabilité du complexe siARN - nanoparticule dans un fluide biologique. Les inventeurs de la présente demande ont observé que le complexe siARN - LNP décrit dans la demande FR 2 994 849 est stable dans un milieu artificiel qui n'est pas physiologique (milieu de culture cellulaire), mais qu'une dissociation du siARN et des LNP se produit dans un milieu complexe reproduisant les conditions physiologiques, en particulier celles du colon distal.

Il existe donc un besoin de fournir un véhicule de siRNA qui soit plus stable en milieu physiologique que les véhicules de l'art antérieur tout en permettant de maintenir l'efficacité biologique, afin de permettre une administration orale, topique, intra rectale ou ophtalmologique. En particulier, une formulation permettant une administration orale est recherchée, ce qui requiert que le véhicule de siRNA puisse franchir les différents milieux intestinaux, avec conditions extrêmes (pH, sels biliaires, enzymes...) pour permettre au siARN d'atteindre sa cible.

A cet effet, selon un premier objet, l'invention concerne une molécule hybride ADN/ARN, appelée htiARN, comprenant :
- deux brins d'ARN antisens de séquences identiques et étant susceptibles de moduler des mécanismes endogènes d'ARN interférence, chacun comprenant un nombre n_{ARN} de nucléotides de 18 à 30, et ayant une extrémité 5' phosphorylée,
- un premier brin d'ADN dont la séquence nucléotidique comprend une séquence nucléotidique S1 liée par son extrémité 3' à l'extrémité 5' d'un bras espaceur nucléotidique L1 lié par son extrémité 3' à l'extrémité 5' d'une séquence nucléotidique SC1, et
- un deuxième brin d'ADN dont la séquence nucléotidique comprend une séquence nucléotidique S2 liée par son extrémité 3' à l'extrémité 5' d'un bras espaceur nucléotidique L2 lié par son extrémité 3' à l'extrémité 5' d'une séquence nucléotidique SC2,
où :
- la séquence nucléotidique SC1 comprend un nombre n_{SC1} nucléotides avec n_{SC1} de (n_{ARN} - 3) à (n_{ARN} + 3), et présente au moins 40 % d'identité de séquence, déterminé par une méthode d'alignement globale, avec la séquence complémentaire de l'ARN antisens, de sorte que la séquence nucléotidique SC1 est hybridée par complémentarité à un premier des deux brins d'ARN antisens,
- la séquence nucléotidique SC2 comprend un nombre n_{SC2} nucléotides avec n_{SC2} de (n_{ARN} - 3) à (n_{ARN} + 3), et présente au moins 40 % d'identité de séquence, déterminé par une méthode d'alignement globale, avec la séquence complémentaire de l'ARN antisens, de sorte que la séquence nucléotidique SC2 est hybridée par complémentarité au deuxième des deux brins d'ARN antisens,
- la séquence nucléotidique S1 du premier brin d'ADN est complémentaire de la séquence nucléotidique S2 du deuxième brin d'ADN, de sorte que la séquence nucléotidique S1 du premier brin d'ADN est hybridée par complémentarité à la séquence nucléotidique S2 du deuxième brin d'ADN,
- les deux séquences nucléotidiques S1 et S2 ont un même nombre n_{S1-S2} de nucléotides, n_{S1-S2} étant un nombre de 16 à 30,
- le bras espaceur nucléotidique L1 du premier brin d'ADN comprend un nombre n_{L1} de nucléotides et le bras espaceur nucléotidique L2 du deuxième brin d'ADN comprend un nombre n_{L2} de nucléotides, n_{L1} et n_{L2} représentant indépendamment un nombre de 1 à 15,
- les deux brins d'ARN antisens, le premier brin d'ADN et/ou le deuxième brin d'ADN sont éventuellement porteurs d'un ou plusieurs groupes lipidiques.

L'invention repose sur la découverte que l'hybride htiARN défini ci-dessus est capable de former avec une nanoparticule un complexe plus stable en milieu physiologique que celui obtenu par complexation de la même nanoparticule avec l'ARN antisens qu'il comprend, tout en conservant une capacité à moduler les mécanismes endogènes d'ARN interférence. En l'occurrence, le complexe hybride htiARN / nanoparticule permet un bon compromis entre stabilité du complexe, ce qui permet d'éviter une décomplexation dans les milieux physiologiques, et efficacité de la modulation des mécanismes endogènes d'ARN interférence. Un tel compromis est difficile à obtenir, car si le complexe est trop stable, l'ARN antisens n'est pas libéré et il ne peut pas moduler les mécanismes endogènes d'ARN interférence, mais si le complexe n'est pas assez stable, l'ARN antisens est libéré prématurément et ne peut pas atteindre sa cible.

Des modifications apportées à la structure d'un siARN ont été décrites dans la littérature, comme l'allongement du brin sens ARN ou son remplacement par de l'ADN. Un allongement de la taille du siARN au-delà de 30 paires de bases entraine une réponse inflammatoire, si bien qu'on évite d'allonger les siARN. De plus, à la connaissance des inventeurs, aucune de ces modifications n'a été utilisée pour réaliser un hybride htiARN tel que défini ci-dessus, ou bien pour stabiliser la complexation d'un médicament ARN sur une nanoparticule.

Sans vouloir être liés à une théorie particulière, les inventeurs supposent que cette stabilisation améliorée du complexe hybride htiARN / nanoparticule pourrait s'expliquer par l'augmentation de la longueur de la molécule d'acide nucléique (n_{SC1} + n_{L1} + n_{S1} + n_{ARN}) par rapport à celle de l'ARN antisens (n_{ARN} de 21 à 23 nucléotides lorsqu'il est monobrin, ou 42 à 46 bases lorsqu'il est double brin), ce qui induirait une charge nette totale plus importante de l'hybride htiARN par rapport à celle de l'ARN antisens, et donc une meilleure affinité de l'hybride htiARN avec la nanoparticule grâce à des forces électrostatiques plus importantes. Cette meilleure affinité expliquerait la meilleure stabilité du complexe hybride htiARN / nanoparticule dans les fluides physiologiques complexes, en prévenant ou limitant sa séparation par compétition avec les différents électrolytes et macromolécules chargées présentes dans un milieu biologique complexe. Cette stabilité renforcée permet ainsi d'augmenter l'efficacité de délivrance d'un siARN dont la séquence antisens est incluse dans la structure du htiARN.

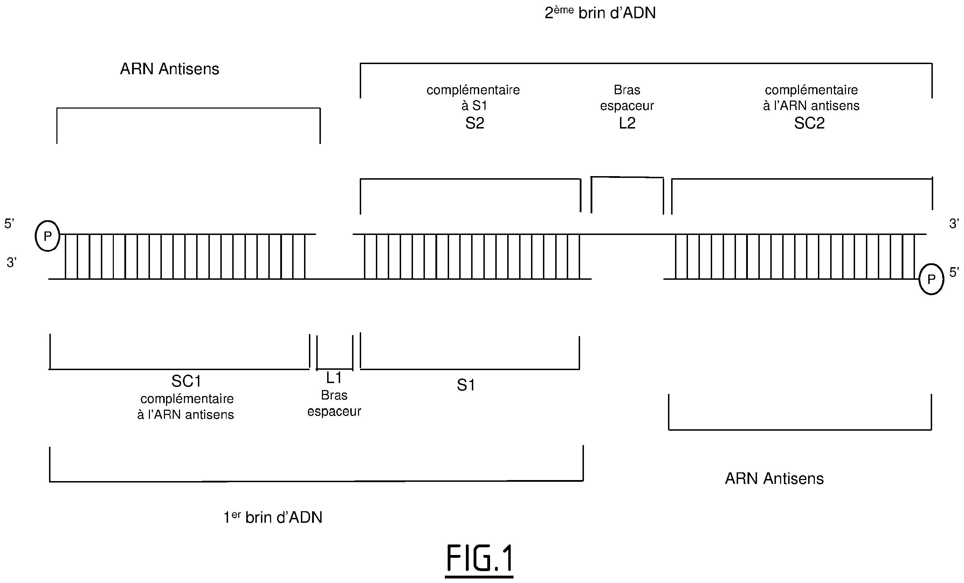

La structure du htiARN est représentée à la [Fig 1] figure 1.

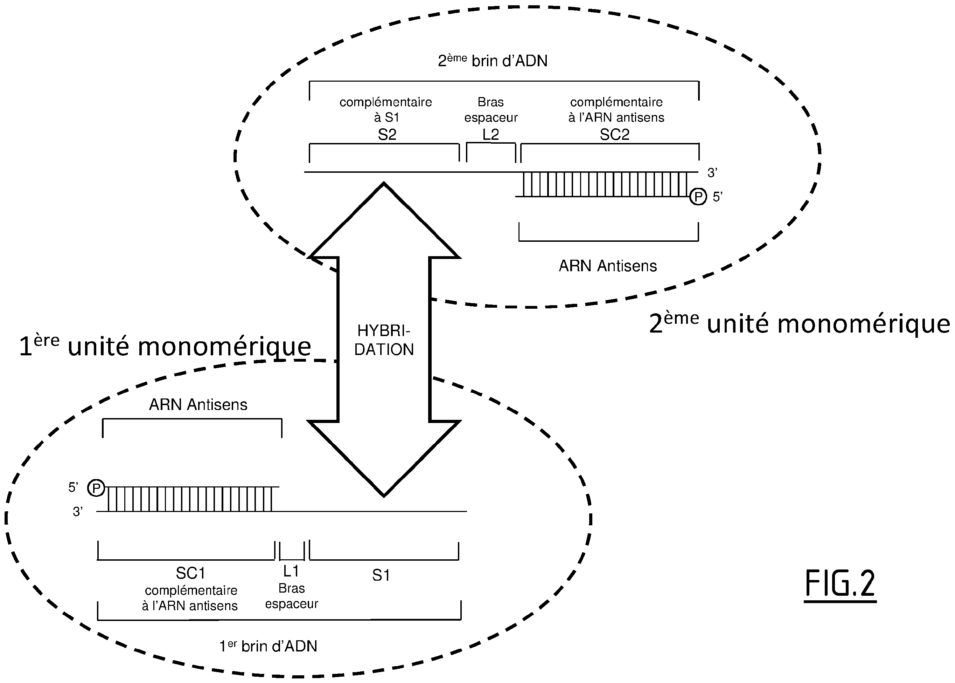

L'hybride htiARN est un hybride d'ARN et d'ADN. L'hybride htiARN comprend quatre brins : deux brins d'ARN et deux brins d'ADN. L'hybride htiARN est un dimère de deux unités monomériques illustrées sur la [Fig 2] figure 2, où chaque unité monomérique comprend :
- un brin d'ARN antisens (qui est identique entre les deux unités monomériques) et
- un brin d'ADN (soit le premier brin, soit le deuxième brin définis ci-dessus) qui comprend :
   - une séquence nucléotidique SC1 ou SC2 au moins partiellement complémentaire à l'ARN antisens, et
   - une séquence nucléotidique S1 ou S2 complémentaire de celle de l'autre unité monomérique (S1 complémentaire de S2 et vice versa).

L'hybride htiARN contient deux ARN antisens, ce qui maximise le nombre de siARN, en comparant par exemple à une structure dans laquelle seul de l'ADN inerte serait ajouté au siARN. Chacun des deux brins d'ARN antisens comprend un nombre n_{ARN} de nucléotides de 18 à 30, notamment de 19 à 27, par exemple de 20 à 25, de préférence de 21 à 23.

Chacune des séquences nucléotidiques SC1 et SC2 peut indépendamment avoir jusque trois nucléotides en moins ou en plus par rapport à la séquence de l'ARN antisens. De plus, les séquences nucléotidiques SC1 et SC2 doivent comporter au moins (40 % d'identité, de préférence au moins 45%, 50%, 55%, 60%, 65%, 70%, 75%, 80%, 85%, 90%, 95% ou 100% d'identité, déterminé par une méthode d'alignement globale, avec la séquence complémentaire de la séquence de l'ARN antisens. Cette complémentarité, partielle ou totale, permet que la séquence SC1 du premier brin d'ADN soit hybridée à un premier ARN antisens et que la séquence SC2 du deuxième brin d'ADN soit hybridée au deuxième ARN antisens. De préférence, SC1 et SC2 sont identiques, ce qui facilite la préparation de l'hybride htiARN.

Un pourcentage d'identité peut être déterminé par une méthode d'alignement globale en utilisant, par exemple, le programme Clustal Oméga (Sievers F et al. (2011). Molecular Systems Biology 7:539 doi:10.1038/msb.2011.75 ; Sievers F, Higgins DG (2018). Protein Sci 27:135-145).

La séquence nucléotidique S1 du premier brin d'ADN et la séquence nucléotidique S2 du deuxième brin d'ADN sont complémentaires l'une de l'autre et permettent l'association par hybridation des deux unités monomériques sous la forme d'un tandem inversé, comme illustré à la [Fig 2] figure 2.

De préférence, la séquence nucléotidique S1-L1 du premier brin d'ADN et/ou la séquence nucléotidique S2-L2 du deuxième brin d'ADN a(ont) moins de 80%, notamment 60%, notamment moins de 50%, typiquement moins de 40%, notamment moins de 30%, de préférence moins de 20%, d'identité avec une séquence ARN du transcriptome (de préférence le transcriptome humain), déterminé par une méthode d'alignement locale. En effet, les séquences nucléotidiques S1 et S2 n'ont de préférence pas de rôle biologique pour éviter un effet indésirable. Le rôle des séquences nucléotidiques S1-L1 et S2-L2 est d'augmenter la charge nette totale de l'hybride htiARN et ainsi d'améliorer la stabilité du complexe hybride htiARN / nanoparticule.

Un pourcentage d'identité peut être déterminé par une méthode d'alignement locale en utilisant, par exemple, le programme Nucléotide BLAST du NCBI (National Center for Biotechnology Information).

Comme transcriptome de référence, il est fait référence en particulier au transcriptome humain généré par le Genome Reference Consortium, en particulier la version GRCh38.p12 du 21 décembre 2017.

Les séquences nucléotidiques S1 et S2 étant complémentaires, elles ont le même nombre n_{S1-S2} de nucléotides. n_{S1-S2} est un nombre entier de 16 à 30. Plus n_{S1-S2} est élevé, plus l'hybridation entre les deux unités monomériques de l'hybride htiARN est forte et plus la charge nette totale de l'hybride htiARN augmente, donc plus sa capacité à stabiliser un complexe hybride htiARN / nanoparticule augmente, mais plus le risque que S1 ou S2 ait un effet biologique non désiré augmente. C'est pourquoi n_{S1-S2} est inférieur ou égal à 30. Lorsque n_{S1-S2} est inférieur à 16, les deux unités monomériques s'hybrident moins bien. Un n_{S1-S2} entre 18 et 22 est optimal pour qu'il y ait une bonne hybridation entre les deux unités monomériques de l'hybride htiARN tout en minimisant le risque d'effet biologique non désiré.

Les deux brins d'ARN antisens sont identiques et chacun comprend de 21 à 23 nucléotides. Leur extrémité 5' est phosphorylée afin de permettre la liaison avec le complexe protéique impliqué dans le processus d'ARN interférence (complexe RISC). L'ARN antisens est susceptible de moduler des mécanismes endogènes d'ARN interférence, et donc susceptible de bloquer l'expression d'un gène cible par le processus d'ARN interférence.

Le bras espaceur nucléotidique L1 sert à relier la séquence nucléotidique S1 et la séquence nucléotidique SC1 du premier brin d'ADN. Le bras espaceur nucléotidique L2 sert à relier la séquence nucléotidique S2 et la séquence nucléotidique SC2 du deuxième brin d'ADN. Les deux bras espaceur peuvent comprendre un nombre de nucléotides n_{L1} et n_{L2} identiques ou différents. Lorsque n_{L1} et n_{L2} sont égaux, les séquences des bras espaceurs nucléotidiques L1 et L2 peuvent être identiques ou différentes. n_{L1} et n_{L2} sont indépendamment un nombre entier de 1 à 15, notamment de 2 à 10, par exemple de 5. Plus n_{L1} ou n_{L2} est élevé, plus le risque que bras espaceur nucléotidique L1 ou L2 ait un effet biologique non désiré augmente. C'est pourquoi n_{L1} et n_{L2} sont inférieurs ou égaux à 15, et sont de préférence le plus faibles possibles.

Un ou les deux brins d'ARN antisens, le premier brin d'ADN et/ou le deuxième brin d'ADN peuvent être éventuellement porteurs d'un ou plusieurs groupes lipidiques. Ce(s) groupe(s) lipidique(s) permet avantageusement de rendre l'hybride htiARN plus lipophile, et de faciliter son passage à travers les membranes plasmiques ou encore renforcer son affinité pour une nanoparticule lipidique. Un groupe cholestérol peut être utilisé comme groupe lipidique. De préférence, le groupe lipidique est greffé sur le premier et/ou deuxième brin d'ADN, par exemple sur leur extrémité 3'.

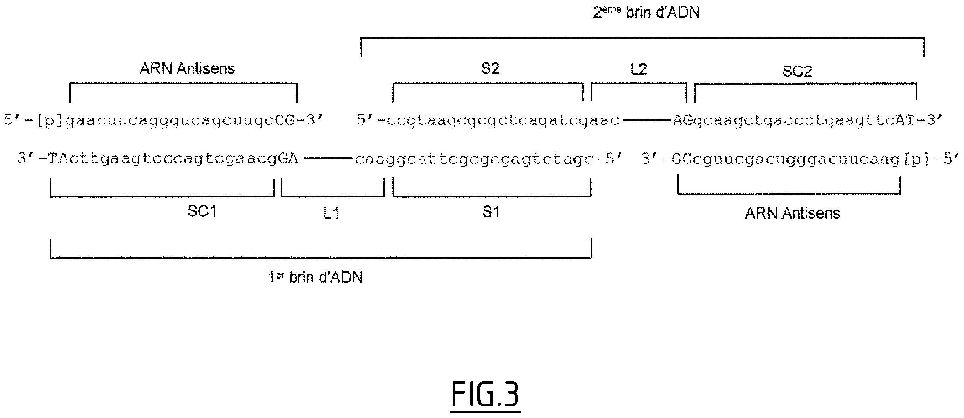

La [Fig. 3] figure 3 fournit un exemple d'hybride htiARN, nommé « htiGFP » car l'ARN antisens qu'il contient est un ARN interférent ciblant l'ARN messager du gène codant pour la « Green Fluorescent Protein » (GFP). Le 1^{er} brin d'ADN a la séquence SEQ ID NO:1. Le 2^{ème} brin d'ADN a la séquence SEQ ID NO:2. Chacun des deux brins d'ARN antisens comprend la séquence SEQ ID NO:3. Chacun contient n_{ARN} = 22 nucléotides. Les séquences nucléotidiques SC1 du premier brin d'ADN et SC2 du deuxième brin d'ADN sont identiques et chacune consiste en la séquence SEQ ID NO:4. Les séquences nucléotidiques SC1 et SC2 contiennent chacune n_{SC1} = n_{SC2} = 22 nucléotides, dont 20 nucléotides consécutifs sont complémentaires de 20 nucléotides consécutifs de SEQ ID NO:3. Les deux nucléotides AT de SEQ ID NO:4 représentent une extrémité débordante en 3' de SC1 et SC2, ces deux nucléotides n'ayant pas de nucléotides complémentaires dans l'ARN antisens. De même, deux nucléotides CG de SEQ ID NO:3 représentent une extrémité débordante en 3' de l'ARN antisens. Le bras espaceur L1 et le bras espaceur L2 sont identiques et consistent chacun en la séquence AACAG. Les bras espaceur L1 et L2 contiennent chacun n_{L1} = n_{L2} = 5 nucléotides. La séquence nucléotidique S1 du premier brin d'ADN consiste en la séquence SEQ ID NO:5. La séquence nucléotidique S2 du deuxième brin d'ADN consiste en la séquence SEQ ID NO:6. Les séquences nucléotidiques S1 et S2 sont complémentaires. Le nombre de nucléotides de chaque séquence nucléotidique S1 et S2 est n_{S1-S2}= 20.

De préférence, la somme n_{SC1} + n_{L1} + n_{S1} + n_{ARN} et/ou la somme n_{SC2} + n_{L2} + n_{S2} + n_{ARN} est(sont) supérieure(s) ou égale(s) à 60 pour une stabilisation optimale du complexe hybride htiARN / nanoparticule.

L'hybride htiARN peut être préparé par hybridation dans un tampon d'hybridation de deux équivalents molaire d'ARN antisens, d'un équivalent molaire de premier brin d'ADN et d'un équivalent molaire de deuxième brin d'ADN.

Selon un deuxième objet, l'invention concerne un complexe d'au moins une nanoparticule et d'au moins un hybride htiARN tel que défini ci-dessus. Ce complexe est également appelé « complexe hybride htiARN / nanoparticule ». Généralement, une nanoparticule est complexée avec plusieurs hybrides htiARN.

La nanoparticule peut être une nanoparticule neutre, anionique ou cationique, de préférence cationique, ce qui favorise les interactions électrostatiques avec l'hybride htiARN.

La nanoparticule est de préférence synthétique. L'utilisation d'une nanoparticule synthétique permet d'effectuer des manipulations peu contraignantes, par rapport à des manipulations mettant en oeuvre des microorganismes ou des virus.

Dans certains modes de réalisation, ladite nanoparticule est une nanoparticule inorganique, comme par exemple une nanoparticule d'or, une nanoparticule magnétique (nanoparticule d'oxyde de fer), ou un nanocristal de matériau semi-conducteur (« quantum dot » en anglais).

Dans certains modes de réalisation, ladite nanoparticule est une nanoparticule organique. Des exemples de nanoparticules utilisables sont notamment décrits dans Morille et al., 2008, Biomaterials, 29 :3477-3496 ; Bruno, 2011, Advanced Drug Delivery Reviews, 63 :1210-1226 ; Zhang et al., 2012, Bioorganic Chemistry, 40 :10-18.

Dans certains modes de réalisation, ladite nanoparticule est une nanoparticule hybride organique/inorganique, typiquement une nanoparticule comportant un coeur inorganique recouvert d'une couche organique, notamment une couche polymérique.

Dans un mode de réalisation, la nanoparticule est une nanoparticule organique comprenant au moins un polymère. Des exemples de polymères sont le Polyéthylèneimine (PEI), la poly(L-lysine) (PLL), le poly(α-[4-amino-butyl]-L-glycolique acide), le chitosan, tel que le chitosan galactosylé, le chitosan-graft-poly(vinylpyrrolidone) (PVP) galactosylé, des oligomères de chitosan trimétylés, le chitosan N-dodécylé, le déoxycholique acide chitosan modifié, la polyamidoamine (PAMAM), le poly(acide lactique) (PLA pour Poly(lactic acid) en anglais), le poly(acide lactique-co-glycolique) (PLGA pour poly(lactic-co-glycolic acid) en anglais), le polyalkylcyanoacrylate (PACA), les dérivés de cyanoacrylate, tels que le polybutylcyanoacrylate (PBCA), le polyisobutylcyanoacrylate (PIBCA), le polyisohexylcyanoacrylate (PIHCA), le polyhexylcyanoacrylate (PHCA), ou l'isobutylcyanoacrylate (IBCA). Optionnellement, ladite nanoparticule peut en outre comprendre au moins un poly(ethylène-glycol) (PEG).

Dans un mode de réalisation, la nanoparticule est une nanoparticule organique cationique comprenant au moins un lipide. Par exemple, la nanoparticule organique cationique comprenant au moins un lipide est un liposome, ou une gouttelette d'une nanoémulsion comprenant une phase aqueuse continue et au moins une phase lipidique dispersée.

De préférence, ledit liposome comprend ou consiste en au moins un lipide aliphatique monovalent, tel que le 1,2-dioleyl-3-trimethylamonium-propane (DOTAP), le *N*[1-(2,3-dioléyloxy) propyl]-*N,N,N*-triméthylammonium (DOTMA), ou le *N*-(2-hydroxyethyl)-*N,N*-dimethyl-2,3-bis(tetradecyloxy-1-propananium) (DMRIE), un lipide aliphatique multivalent, tel que le dioctadecylamidoglycylspermine (DOGS), ou un dérivé cationique du cholestérol, tel que le 3β-[N-(*N',N'-*diméthyl-aminoéthane)-carbamoyl]cholestérol (DC-Chol) ou le bis-guanidium-tren-cholestérol (BGTC). Optionnellement, ledit liposome peut comprendre en outre un lipide fusogène, qui est susceptible de faciliter la libération cytosolique par déstabilisation de la membrane endosomale, dit lipide « helper », tel que le dioléylphosphatidyléthanolamine (DOPE) ou le cholestérol. Ledit liposome peut comprendre au moins 2, 3, 4, 5 lipides tels que décrits ci-dessus. A titre d'exemples non limitatifs, ledit liposome peut donc être un liposome DOTMA/DOPE ou DOTAP/cholestérol. Optionnellement, ledit liposome peut en outre comprendre au moins un poly-ethylène-glycol (PEG). Des exemples de liposomes comprenant en outre au moins un PEG sont des liposomes constitués de *N,N*-dioleyl-*N,N-*dimethylammonium chloride, de DOPE et de céramides conjugués un PEG, des liposomes constitués de 3-*N*-[methoxypoly(ethyleneglycol)₂₀₀₀)carbamoyl]-1,2dimyristoyloxy-propylamine (PEG-C-DMA), 1,2-dilinoleylloxy-*N*-*N*-dimethym-3-aminopropane (DLinDMA), 1,2-distearoyl-sn-glycero-3-phosphocholine (DSPC) et de cholestérol à un rapport 2:40:10:48 pour cent molaires, des liposomes constitués de β-L-arginyl-2,3-L-diaminopropionic acid-N-palmityl-N-oleyl-amide trihydrochloride, 1,2-diphytanoyl-sn-glycero-3-phosphoethanolamine (DphyPE et le lipide PEGylé N-(carbonyl methyxypolyethyleneglycol-2000)-1,2-distearoyl-sn-glycero-3-phospho-ethanolamine sodium (DSPE-PEG), des liposomes constitués de phosphatidyl choline hydrogénée de soja, de cholestérol, de DSPE-PEG2000 et de DOTAP.

De préférence, ladite gouttelette d'une nanoémulsion, comprenant une phase aqueuse continue et au moins une phase dispersée, est telle que définie dans la demande FR 2 994 849. Ce mode de réalisation est particulièrement préféré et est détaillé ci-après.

Le complexe hybride htiARN / nanoparticule est alors sous forme d'une nanoémulsion comprenant une phase aqueuse continue et au moins une phase dispersée qui comprend :
a) au moins 5% molaire de lipide amphiphile,
b) de 15 à 70% molaire d'au moins un tensioactif cationique comprenant :
   i) au moins un groupe lipophile choisi parmi :
      - un groupe R ou R-(C=O)-, où R représente une chaîne hydrocarbonée linéaire comprenant de 11 à 23 atomes de carbone,
      - un ester ou un amide d'acides gras comprenant de 12 à 24 atomes de carbone et de phosphatidyléthanolamine, et
      - un poly(oxyde de propylène), et
   ii) au moins un groupe hydrophile comprenant au moins un groupe cationique choisi parmi :
      - un groupe alkyle linéaire ou ramifié comprenant de 1 à 12 atomes de carbone et interrompu et/ou substitué par au moins un groupe cationique, et
      - un groupe polymérique hydrophile comprenant au moins un groupe cationique, et
c) de 10% à 55% molaire d'un co-tensioactif comprenant au moins une chaîne poly(oxyde d'éthylène) comprenant au moins 25 unités d'oxyde d'éthylène,
d) un lipide solubilisant comprenant au moins un glycéride d'acides gras,
e) éventuellement un lipide fusogène,
f) un hybride htiARN tel que défini ci-dessus,
où les pourcentages molaires de lipide amphiphile, de tensioactif cationique et de co-tensioactif sont par rapport à l'ensemble (lipide amphiphile / tensioactif cationique / co-tensioactif / éventuel lipide fusogène).

Comme expliqué ci-après, les : lipide amphiphile / tensioactif cationique / co-tensioactif / éventuel lipide fusogène / hybride htiARN sont les principaux composants de la couronne des gouttelettes de la nanoémulsion.

La nanoémulsion est donc une émulsion de type huile dans l'eau. Elle peut être simple ou multiple, notamment en comportant dans la phase dispersée une seconde phase aqueuse. De préférence, elle est simple.

Un ou plusieurs hybrides htiARN (généralement plusieurs) est(sont) complexé(s) à la surface des gouttelettes de ladite nanoémulsion.

### Tensioactif cationique

La nanoémulsion utilisée comprend un tensioactif cationique comprenant :
a) au moins un groupe lipophile choisi parmi :
   i) un groupe R représentant une chaîne hydrocarbonée linéaire comprenant de 11 à 23 atomes de carbone,
   ii) un ester ou un amide d'acides gras comprenant de 12 à 24 atomes de carbone et de phosphatidyléthanolamine, tel que le distéaryl phosphatidyléthanolamine (DSPE), et
   iii) un poly(oxyde de propylène), et
b) au moins un groupe hydrophile comprenant au moins un groupe cationique choisi parmi :
   i) un groupe alkyle linéaire ou ramifié comprenant de 1 à 12 atomes de carbone et interrompu et/ou substitué par au moins un groupe cationique, et
   ii) un groupe polymérique hydrophile comprenant au moins un groupe cationique, ledit groupe polymérique étant notamment choisi parmi :
      - un poly(oxyde d'éthylène) comprenant typiquement de 3 à 500 unités oxyde d'éthylène, de préférence de 20 à 200 unités oxyde d'éthylène, et comprenant au moins un groupe cationique.
      - un polysaccharide, tel que du dextrane, de la cellulose ou du chitosan, ayant notamment des masses moléculaires comprises entre 0,5 et 20 kDa, par exemple entre 1 et 12 kDa,
      - un polyamine, tel qu'un chitosan ou une polylysine, ayant notamment des masses moléculaires comprises entre 0,5 et 20 kDa, par exemple entre 1 et 12 kDa.

Par «ester ou amide d'acides gras comprenant de 12 à 24 atomes de carbone et de phosphatidyléthanolamine», on entend un groupe de formule : 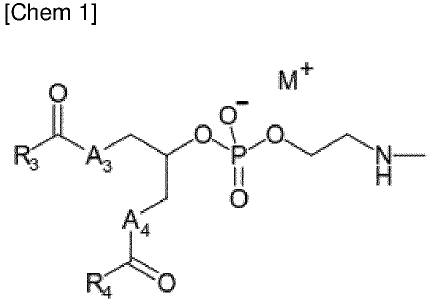 dans laquelle
- R₃ et R₄ représentent indépendamment une chaîne hydrocarbonée linéaire comprenant de 11 à 23 atomes de carbone,
- A₃ et A₄ représentent O ou NH, et
- M représente H ou un cation.

Les groupes cationiques du tensioactif cationique sont typiquement :
- des oniums choisis parmi les groupes ammonium, imidazolium, pyridinium, pyrrolidinium, pipéridinium, phosphonium ou sulfonium, ou
- des complexes métalliques entre un radical d'un groupe organique chélatant mono- ou multi-dentate, par exemple la phénantroline, la pyridine, l'acide éthylène diamine tétraacétique (EDTA), l'acide diéthylène triamine penta acétique (DTPA), les porphyrines, les phtalocyanines, les clorines, les bactériochlorines complexé avec un cation inorganique, tel que Ca²⁺, Al³⁺, Ni⁺, Zn²⁺, Fe²⁺, Fe³⁺ ou Cu²⁺,
les groupes ammonium, notamment -⁺NMe₃, -⁺NHMe₂, -⁺NH₂Me et -⁺NH₃, en particulier -⁺NH₃, étant particulièrement préféré.

Bien sûr, des anions peuvent être associés au(x) groupe(s) cationique(s) pour que la nanoémulsion soit électriquement neutre. La nature des anions n'est pas limitée. A titre illustratif, on peut citer les halogénures, notamment le chlorure ou le bromure, ou le trifluoroacétate.

Dans le tensioactif cationique, la nature du groupe de liaison liant le(s) groupe(s) lipophile(s) au(x) groupe(s) hydrophile(s) comprenant au moins un groupe cationique n'est pas limitée. Des exemples de groupes de liaison sont fournis ci-dessous (groupe L).

Le tensioactif cationique peut avoir la formule (A) suivante :

[(Lipo)ₗ-L-(Hydro)ₕ]ⁿ⁺, (n/m) [A]^{m-} (A)

dans laquelle:
- l et h représentent des nombres entiers indépendamment compris entre 1 et 4,
- n est un nombre entier supérieur ou égal à 1, généralement compris entre 1 et 50,
- Lipo représente un groupe lipophile tel que défini ci-dessus,
- Hydro représente un groupe hydrophile tel que défini ci-dessus comprenant au moins un groupe cationique,
- L représente un groupe de liaison,
- A représente un anion,
- m est un nombre entier représentant la charge de l'anion,
- n est un nombre entier représentant la charge du cation [(Lipo)ₗ-L-(Hydro)ₕ].

Dans la formule (A) susmentionnée, de préférence L est tel que :
a) lorsque l et h représentent 1, L est un groupe de liaison divalent choisi parmi :
   - une liaison simple,
   - un groupe Z choisi parmi -O-, -NH-, -O(OC)-, -(CO)O-, -(CO)NH-, -NH(CO)-, -O-(CO)-O-, -NH-(CO)-O-, -O-(CO)-NH- et -NH-(CO)-NH, -O-PO(OH)-O- ou un radical divalent cyclique de 5 à 6 atomes,
   - un groupe Alk étant un alkylène comprenant de 1 à 6 atomes de carbone, et
   - un groupe Z-Alk, Alk-Z, Alk-Z-Alk ou Z-Alk-Z où Alk et Z sont tels que définis ci-dessus et où les deux groupes Z du groupe Z-Alk-Z sont identiques ou différents,
b) lorsque l'un des groupes l ou h représente 1, et l'autre représente 2, L est un groupe trivalent choisi parmi un groupe phosphate OP-(O-)₃, un groupe dérivé du glycérol de formule -O-CH₂-CH-(O-)CH₂-O- et un radical trivalent cyclique de 5 à 6 atomes,
   - pour les autres valeurs de l et h, L est un radical multivalent cyclique de 5 à 6 atomes.

De manière particulièrement préférée, L est tel que :
a) lorsque l et h représentent 1, L est un groupe de liaison divalent choisi parmi :
   - une liaison simple,
   - un groupe Z choisi parmi -O-, -NH-, -O(OC)-, -(CO)O-, -(CO)NH-, -NH(CO)-, -O-(CO)-O-, -NH-(CO)-O-, -O-(CO)-NH- et -NH-(CO)-NH ou -O-PO(OH)-O-,
b) lorsque l'un des groupes l ou h représente 1, et l'autre représente 2, L est un groupe trivalent choisi parmi un groupe phosphate OP-(O-)₃ et un groupe dérivé du glycérol de formule -O-CH₂-CH-(O-)CH₂-O-.

Dans la formule (A) susmentionnée, l et h représentent de préférence indépendamment 1 ou 2.

Selon une première alternative, le groupe hydrophile du tensioactif cationique est un groupe alkyle linéaire ou ramifié comprenant de 1 à 12 atomes de carbone et interrompu et/ou substitué par au moins un groupe cationique. Comme exemple de tels tensioactifs cationiques, on peut citer:
1) (Lipo)-(CH₂)ₘ₁-NR₃₀R₃₁R₃₂, où Lipo est un groupe lipophile tel que défini ci-dessus, m1 représente 1 ou 2 et R₃₀, R₃₁ et R₃₂ représentent indépendamment H, Me ou -CH₂-CH₂-OH,
2) 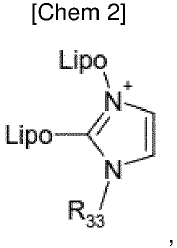 où chaque Lipo est indépendamment un groupe lipophile tel que défini ci-dessus, et R33 représente H, Me ou -CH₂-CH₂-OH,
3) 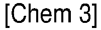 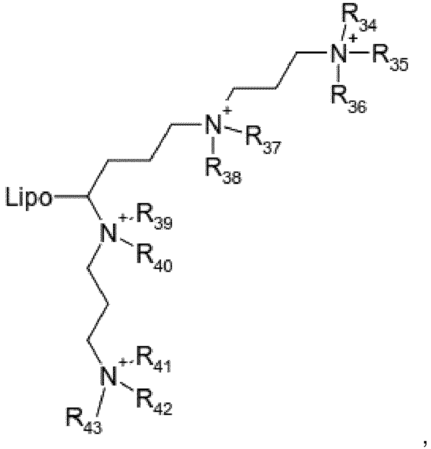 où Lipo est un groupe lipophile tel que défini ci-dessus, et R₃₄, R₃₅, R₃₆, R₃₇, R₃₈, R₃₉, R₄₀, R₄₁, R₄₂ et R₄₃ représentent indépendamment H, Me ou -CH₂-CH₂-OH.

De préférence, le tensioactif cationique est choisi parmi :
- le *N*[1-(2,3-dioléyloxy) propyl]-*N,N,N*-triméthylammonium (DOTMA),
- le 1,2-dioleyl-3-trimethylamonium-propane (DOTAP),
- le *N*-(2-hydroxyethyl)-*N,N*-dimethyl-2,3-bis(tetradecyloxy-1-propananium) (DMRIE),
- le 1-[2-(oleoyloxy)ethyl]-2-oleyl-3-(2-hydroxyethyl)imidazolinium (DOTIM), et
- le dioctadecylamidoglycylspermine (DOGS) (sous forme protonée),
et est de préférence le 1,2-dioleyl-3-trimethylamonium-propane (DOTAP).

Selon une deuxième alternative, le groupe hydrophile du tensioactif cationique est un groupe polymérique hydrophile comprenant au moins un groupe cationique.

Lorsque le groupe hydrophile du tensioactif cationique est polymérique, le(s) groupe(s) cationique(s) peu(ven)t être un(des) groupe(s) terminal(aux) ou pendant(s). Par exemple :
- lorsque groupe polymérique hydrophile est un poly(oxyde d'éthylène), le(s) groupe(s) cationique(s) est(sont) généralement situé(s) sur un groupe terminal à l'extrémité de la chaîne poly(oxyde d'éthylène).
- lorsque le groupe polymérique hydrophile est du dextrane ou de la cellulose, le(s) groupe(s) cationiques est(sont) généralement situé(s) sur un groupe terminal à l'extrémité de la chaîne polysaccharidique.
- lorsque le groupe polymérique hydrophile est du chitosan, le(s) groupe(s) cationiques est(sont) généralement un(des) groupe(s) pendant(s), en particulier des groupes -NH₃⁺ présents en milieu acide sur le chitosan.

Dans un mode de réalisation, le(s) groupe(s) cationique(s) est(sont) un(des) groupe(s) terminal(aux). En effet, les groupes pendants de tensioactifs anioniques adjacents en surface des gouttelettes de la phase dispersée se repoussent par interactions électrostatiques et, par conséquent, les nanoémulsions comprenant des tensioactifs cationiques dont le groupe Hydro comprend des groupes pendants sont généralement moins stables.

Dans un autre mode de réalisation, le(s) groupe(s) cationique(s) est(sont) un(des) groupe(s) pendant(s). Il est avantageusement possible d'utiliser un tensioactif cationique dont le groupe hydrophile comprend plusieurs groupes cationiques pendants, et donc d'obtenir une nanoémulsion plus chargée positivement, et donc dans laquelle l'hybride htiARN est complexé plus fortement aux gouttelettes.

Le groupe polymérique hydrophile préféré est un radical d'un poly(oxyde d'éthylène) comprenant typiquement de 3 à 500 unités oxyde d'éthylène, de préférence de 20 à 200 unités oxyde d'éthylène, et comprenant au moins un groupe cationique.

Ainsi, dans un mode de réalisation, le tensioactif cationique a l'une des formules suivantes : 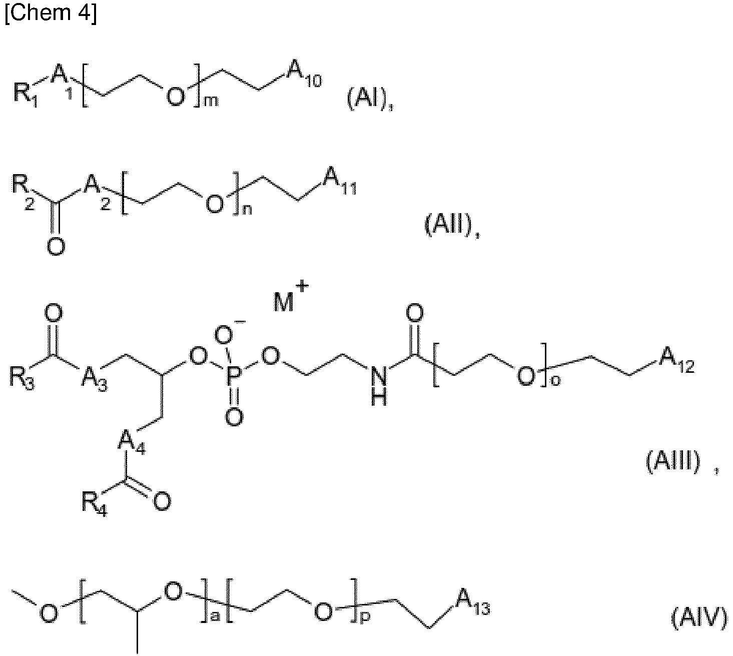 dans lesquelles :
- R₁, R₂, R₃ et R₄ représentent indépendamment une chaîne hydrocarbonée linéaire comprenant de 11 à 23 atomes de carbone,
- A₁, A₂, A₃ et A₄ représentent O ou NH,
- m, n, o et p représentent indépendamment des nombres entiers de 3 à 500, de préférence 20 à 200, et
- a représente un nombre entier de 20 à 120,
- M représente H ou un cation,
- A₁₀, A₁₁, A₁₂ et A₁₃ représentent indépendamment un groupe -⁺NR₂₀R₂₁R₂₂, dans lequel R₂₀, R₂₁ et R₂₂ représentent indépendamment H, Me ou -CH₂-CH₂-OH.

Dans un mode de réalisation, dans la formule (All), A₁₁ représente -⁺NH₃ et le tensioactif cationique a la formule suivante : 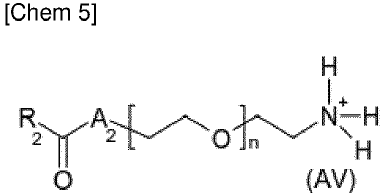 dans laquelle A₂, R₂ et n sont tels que définis ci-dessus. De préférence, dans la formule (All), R₂ représente C₁₇H₃₅.

Sans vouloir être liés à une théorie particulière, la présence du groupe polymérique hydrophile permettrait :
- de stabiliser la nanoémulsion, et
- de protéger le htiARN, localisé à la surface des gouttelettes, des protéines du milieu dans lequel la nanoémulsion est administrée/utilisée, et donc la dégradation de l'hybride htiARN et de l'ARN antisens qu'il contient, par ces protéines.

Selon une troisième alternative, la nanoémulsion comprend au moins deux tensioactifs cationiques, dont :
a) l'un est choisi parmi :
   - le *N*[1-(2,3-dioléyloxy) propyl]-*N,N,N*-triméthylammonium (DOTMA),
   - le 1,2-dioleyl-3-trimethylamonium-propane (DOTAP),
   - le *N*-(2-hydroxyethyl)-*N,N*-dimethyl-2,3-bis(tetradecyloxy-1-propananium) (DMRIE),
   - le 1-[2-(oleoyloxy)ethyl]-2-oleyl-3-(2-hydroxyethyl)imidazolinium chloride (DOTIM), et
   - le dioctadecylamidoglycylspermine (DOGS),
   et est de préférence le 1,2-dioleyl-3-trimethylamonium-propane (DOTAP), et
b) l'autre est un tensioactif cationique comprenant :
   i) au moins un groupe lipophile choisi parmi :
      - un groupe R ou R-(C=O)-, où R représente une chaîne hydrocarbonée linéaire comprenant de 11 à 23 atomes de carbone,
      - un ester ou un amide d'acides gras comprenant de 12 à 24 atomes de carbone et de phosphatidyléthanolamine, tel que le distéaryl phosphatidyléthanolamine (DSPE), et
      - un poly(oxyde de propylène), et
   ii) un groupe polymérique hydrophile comprenant au moins un groupe cationique, ledit groupe polymérique étant choisi parmi :
      - un poly(oxyde d'éthylène) comprenant typiquement de 3 à 500 unités oxyde d'éthylène, de préférence de 20 à 200 unités oxyde d'éthylène, et comprenant au moins un groupe cationique,
      - un polysaccharide, tel que du dextrane, de la cellulose ou du chitosan,
      - un polyamine, tel qu'un chitosan ou une polylysine,
      et est de préférence un poly(oxyde d'éthylène) comprenant au moins un groupe cationique.

Dans un mode de réalisation, la nanoémulsion comprend, à titre de tensioactifs cationiques :
a) du 1,2-dioleyl-3-trimethylamonium-propane, et
b) un tensioactif cationique comprenant :
   i) au moins un groupe lipophile choisi parmi :
      - un groupe R ou R-(C=O)-, où R représente une chaîne hydrocarbonée linéaire comprenant de 11 à 23 atomes de carbone,
      - un ester ou un amide d'acides gras comprenant de 12 à 24 atomes de carbone et de phosphatidyléthanolamine, tel que le distéaryl phosphatidyléthanolamine (DSPE), et
   ii) un poly(oxyde d'éthylène) comprenant typiquement de 3 à 500 unités oxyde d'éthylène, de préférence de 20 à 200 unités oxyde d'éthylène, et comprenant au moins un groupe cationique.

Dans un mode de réalisation, la nanoémulsion comprend, à titre de tensioactifs cationiques :
- du 1,2-dioleyl-3-trimethylamonium-propane, et
- un composé de formule (All) telle que définie ci-dessus, notamment de formule (AV).

Le tensioactif cationique se situe dans la couronne des gouttelettes de la nanoémulsion. Il se lie par interactions électrostatiques à l'hybride htiARN et permet de le maintenir à la surface des gouttelettes. Ce sont ces interactions électrostatiques qui permettent la formation et la stabilité du complexe hybride htiARN / gouttelettes de la nanoémulsion.

La nanoémulsion comprend de 15 à 70% molaire d'au moins un tensioactif cationique par rapport à l'ensemble (lipide amphiphile / tensioactif cationique / co-tensioactif / éventuel lipide fusogène). En dessous de 15%, la nanoémulsion ne comprend pas assez de charges positives et la complexation des gouttelettes avec l'hybride htiARN est insuffisante. Au-delà de 70%, les nanoémulsions ne sont pas stables, et ne peuvent généralement même pas être formulées (la formation de la nanoémulsion n'est pas possible car les gouttelettes coalescent pour former deux phases), et les gouttelettes deviennent généralement toxiques pour les cellules.

Ces proportions sont particulièrement adaptées pour obtenir une complexation efficace de l'hybride htiARN à la surface des gouttelettes, et donc une bonne délivrance et/ou transfection.

### Lipide fusogène (« helper lipid » en anglais)

La nanoémulsion peut comprendre un lipide fusogène, qui est susceptible de faciliter la libération cytosolique par déstabilisation de la membrane endosomale, dit lipide « helper ». De préférence, ce lipide est le dioléylphosphatidyléthanolamine (DOPE).

Le lipide fusogène permet de favoriser l'échappement endosomal des gouttelettes de la nanoémulsion, et donc des hybrides htiARN, qu'elles contiennent, et améliore généralement leur efficacité. Ainsi, le lipide fusogène améliore l'efficacité d'extinction génique de l'hybride htiARN.

Le lipide susceptible de faciliter la libération cytosolique par déstabilisation de la membrane endosomale se situe dans la couronne des gouttelettes de la nanoémulsion.

### Lipide amphiphile

La nanoémulsion comprend au moins un lipide amphiphile, qui se situe dans la couronne des gouttelettes de la nanoémulsion.

Afin de former une nanoémulsion stable, il est nécessaire d'inclure dans la composition au moins un lipide amphiphile à titre de tensioactif. La nature amphiphile du tensioactif assure la stabilisation des gouttelettes d'huile au sein de la phase continue aqueuse. En dessous de 5% molaire de lipide amphiphile par rapport à l'ensemble (lipide amphiphile / tensioactif cationique / co-tensioactif / éventuel lipide fusogène), les nanoémulsions ne sont pas stables, et ne peuvent généralement même pas être formulées (la formation de la nanoémulsion n'est pas possible car les gouttelettes coalescent pour former deux phases).

Généralement, la nanoémulsion comprend de 5 à 85% molaire, de préférence de 5 à 75% molaire, en particulier de 5 à 50% molaire et tout particulièrement de 8 à 30% molaire de lipide amphiphile par rapport à l'ensemble (lipide amphiphile / tensioactif cationique / co-tensioactif / éventuel lipide fusogène).

La quantité de lipide amphiphile contribue avantageusement à contrôler la taille de la phase dispersée de la nanoémulsion.

Les lipides amphiphiles comportent une partie hydrophile et une partie lipophile. Ils sont généralement choisis parmi les composés dont la partie lipophile comprend une chaîne saturée ou insaturée, linéaire ou ramifiée, ayant de 8 à 30 atomes de carbone. Ils peuvent être choisis parmi les phospholipides, les cholestérols, les lysolipides, les sphingomyélines, les tocophérols, les glucolipides, stéarylamines, les cardiolipines d'origine naturelle ou synthétique ; les molécules composées d'un acide gras couplé à un groupement hydrophile par une fonction éther ou ester tels que les esters de sorbitan comme par exemple les monooléate et monolaurate de sorbitan vendus sous les dénominations Span^{®} par la société Sigma; les lipides polymérisés ; les lipides conjugués à de courtes chaînes d'oxyde de polyéthylène (PEG) tels que les tensioactifs non-ioniques vendus sous les dénominations commerciales Tween^{®} par la société ICI Americas, Inc. et Triton^{®} par la société Union Carbide Corp.; les esters de sucre tels que les mono- et di-laurate, mono- et di-palmitate, mono- et distéarate de saccharose; lesdits tensioactifs pouvant être utilisés seuls ou en mélanges.

Les phospholipides sont les lipides amphiphiles préférés.

La lécithine est le lipide amphiphile particulièrement préféré.

### Lipide solubilisant

La nanoémulsion comprend par ailleurs un lipide solubilisant comprenant au moins un glycéride d'acides gras, qui se situe dans la phase dispersée de la nanoémulsion, plus précisément dans le coeur des gouttelettes. Ce composé a pour mission principale de solubiliser le lipide amphiphile, peu soluble, dans la phase dispersée de la nanoémulsion.

Le lipide solubilisant est un lipide présentant une affinité avec le lipide amphiphile suffisante pour permettre sa solubilisation. De préférence, le lipide solubilisant est solide à température ambiante (250°C).

Dans le cas où le lipide amphiphile est un phospholipide, il peut s'agir notamment :
- d'esters d'acides gras et d'alcool gras, comme le cétylpalmitate, ou
- de dérivés du glycérol, et en particulier de glycérides obtenues par estérification de glycérol avec des acides gras.

Le lipide solubilisant utilisé est avantageusement choisi en fonction du lipide amphiphile utilisé. Il présentera généralement une structure chimique proche, afin d'assurer la solubilisation recherchée. Il peut s'agir d'une huile ou d'une cire. De préférence, le lipide solubilisant est solide à température ambiante (20°C), mais liquide à la température du corps (37°C).

Les lipides solubilisants préférés, en particulier pour les phospholipides, sont les esters d'acides gras et d'alcool gras, comme le cétylpalmitate, ou les glycérides d'acides gras, notamment d'acides gras saturés, et en particulier les glycérides d'acides gras saturés comportant 8 à 18 atomes de carbone, encore préféré 12 à 18 atomes de carbone. Avantageusement, le lipide solubilisant est un mélange de différents glycérides.

De préférence, le lipide solubilisant est un mélange s'agit de glycérides d'acides gras saturés comportant au moins 10% en poids d'acides gras en C12, au moins 5% en poids d'acides gras en C14, au moins 5% en poids d'acides gras en C16 et au moins 5% en poids d'acides gras en C18.

De préférence, le lipide solubilisant est un mélange de glycérides d'acides gras saturés comportant 0% à 20% en poids d'acides gras en C8, 0% à 20% en poids d'acides gras en C10, 10% à 70% en poids d'acides gras en C12, 5% à 30% en poids d'acides gras en C14, 5% à 30% en poids d'acides gras en C16 et 5% à 30% en poids d'acides gras en C18.

Particulièrement préférés sont les mélanges de glycérides semi-synthétiques solides à température ambiante (25°C) vendus sous la dénomination commerciale Suppocire^{®}NC par la société Gattefossé et approuvé pour l'injection chez l'homme. Les Suppocire^{®} de type N sont obtenues par estérification directe d'acides gras et de glycérol. Il s'agit de glycérides hémi-synthétiques d'acides gras saturés de C8 à C18, dont la composition quali-quantitative est indiquée dans le tableau 1 ci-dessous.

Les lipides solubilisants précités permettent d'obtenir une nanoémulsion avantageusement stable. Sans vouloir être lié à une théorie particulière, il est supposé que les lipides solubilisants précités permettent d'obtenir des gouttelettes dans la nanoémulsion présentant un coeur amorphe. Le coeur ainsi obtenu présente une viscosité interne élevée sans pour autant présenter de cristallinité. Or, la cristallisation est néfaste pour la stabilité de la nanoémulsion car elle conduit généralement à une agrégation des gouttelettes et/ou à une expulsion des molécules encapsulées à l'extérieur des gouttelettes. Ces propriétés physiques favorisent la stabilité physique de la nanoémulsion.

La quantité de lipide solubilisant peut varier largement en fonction de la nature et de la quantité de lipide amphiphile présent dans la phase dispersée. Généralement, le coeur des gouttelettes (comprenant le lipide solubilisant, l'éventuelle huile, l'éventuel agent d'imagerie, l'éventuel agent thérapeutique s'il est lipophile) comprend de 1 à 100% en poids, de préférence de 5 à 80% en poids et tout particulièrement de 40 à 75% en poids de lipide solubilisant.

### [Tableau 1]

**Tableau 1 : Composition en acides gras du Suppocire^{®} NC de Gattefossé**

| Longueur de chaînes | [% en poids] |
|---|---|
| C8 | 0,1 à 0,9 |
| C10 | 0,1 à 0,9 |
| C12 | 25 à 50 |
| C14 | 10 à 24,9 |
| C16 | 10 à 24,9 |
| C18 | 10 à 24,9 |

### Huile

La phase dispersée de la nanoémulsion peut comporter par ailleurs une ou plusieurs autres huiles, qui se situe(nt) dans le coeur des gouttelettes.

Les huiles utilisées présentent de préférence une balance hydrophile-lipophile (HLB) inférieure à 8 et encore plus préférentiellement comprise entre 3 et 6. Avantageusement, les huiles sont utilisées sans modification chimique ou physique préalablement à la formation de l'émulsion.

Les huiles sont généralement choisies parmi les huiles biocompatibles, et en particulier parmi les huiles d'origine naturelle (végétale ou animale) ou synthétique. Parmi de telles huiles, on peut notamment citer les huiles d'origine naturelle végétale parmi lesquelles figurent notamment les huiles de soja, de lin, de palme, d'arachide, d'olives, de pépin de raisins et de tournesol ; les huiles synthétiques parmi lesquelles figurent notamment les triglycérides, di glycérides et les mono glycérides. Ces huiles peuvent être de premières expressions, raffinées ou inter-estérifiées.

Les huiles préférées sont l'huile de soja et l'huile de lin.

Généralement, si présente, l'huile sera contenue dans le coeur des gouttelettes (comprenant le lipide solubilisant, l'éventuelle huile, l'éventuel agent d'imagerie, l'éventuel agent thérapeutique s'il est lipophile) dans une proportion allant de 1 à 80% en poids, de préférence entre 5 et 50 % en poids et tout particulièrement 10 à 30% en poids.

La phase dispersée peut contenir en outre d'autres additifs tels que des colorants, stabilisants, conservateurs ou d'autres principes actifs, en quantité appropriée.

### Co-tensioactif

La nanoémulsion comprend un co-tensioactif, qui permet de la stabiliser.

Les co-tensioactifs sont généralement des tensioactifs hydrosolubles. Ils comprennent au moins une chaîne poly(oxyde d'éthylène) comprenant au moins 25, notamment au moins 30, de préférence au moins 35, unités d'oxyde d'éthylène. Le nombre d'unités d'oxyde d'éthylène est généralement inférieur à 500.

Des nanoémulsions comprenant un co-tensioactif comprenant une chaîne poly(oxyde d'éthylène) comprenant moins de 25 unités d'oxyde d'éthylène ne sont en effet pas stables. Généralement, il n'est même pas possible de préparer la nanoémulsion.

Ces nombres d'unités sont en effet particulièrement adaptés pour éviter la fuite de l'hybride htiARN hors des gouttelettes.

En effet, les inventeurs ont observé qu'une nanoémulsion ne comprenant pas un co-tensioactif n'est pas suffisamment stable.

De plus, sans vouloir être liés à une théorie particulière, la présence de la chaîne composée de motifs d'oxyde d'éthylène du co-tensioactif permettrait de protéger les hybrides htiARN, localisés à la surface des gouttelettes des protéines du milieu dans lequel la nanoémulsion est administrée/utilisée, et donc la dégradation desdits hybrides htiARN par ces protéines.

A titre d'exemple de co-tensioactifs, on peut en particulier citer les composés conjugués polyéthylèneglycol/phosphatidyl-éthanolamine (PEG-PE), les éthers d'acide gras et de polyéthylèneglycol tels que les produits vendus sous les dénominations commerciales Brij^{®} (par exemple Brij^{®} 35, 58, 78 ou 98) par la société ICI Americas Inc., les esters d'acide gras et de polyéthylèneglycol tels que les produits vendus sous les dénominations commerciales Myrj^{®} par la société ICI Americas Inc. (par exemple Myrj^{®} 45, 52, 53 ou 59) et les copolymères blocs d'oxyde d'éthylène et d'oxyde de propylène tels que les produits vendus sous les dénominations commerciales Pluronic^{®} par la société BASF AG (par exemple Pluronic^{®} F68, F127, L64, L61, 10R4, 17R2, 17R4, 25R2 ou 25R4 ) ou les produits vendus sous la dénomination commerciale Synperonic^{®} par la société Unichema Chemie BV (par exemple Synperonic^{®} PE/F68, PE/L61 ou PE/L64).

Ainsi, le co-tensioactif se situe à la fois dans la phase aqueuse continue et dans la phase dispersée. En effet, la partie hydrophobe du co-tensioactif s'insère dans les gouttelettes de la phase dispersée, alors que les chaînes polyalcoxylées sont dans la phase aqueuse continue. Dans la présente demande, les pourcentages massiques de phase dispersée décrits sont calculés en considérant que le co-tensioactif appartient à la phase dispersée.

La nanoémulsion comprend de 10% à 55% molaire de co-tensioactif par rapport à l'ensemble (lipide amphiphile / tensioactif cationique / co-tensioactif / éventuel lipide fusogène). En dessous de 10%, les nanoémulsions ne sont pas stables, et ne peuvent généralement même pas être formulées (la formation de la nanoémulsion n'est pas possible car les gouttelettes coalescent pour former deux phases). Au-delà de 55%, la complexation des hybrides htiARN sur les gouttelettes de la nanoémulsion n'a pas lieu, probablement car les charges positives du tensioactif cationique sont masquées par les chaînes poly(oxyde d'éthylène) du co-tensioactif, et donc plus accessibles pour se lier par liaison électrostatique aux hybrides htiARN.

### Agent dimagerie

La nanoméulsion peut comprendre un agent d'imagerie, qui permet avantageusement de visualiser la distribution des gouttelettes dans les cellules ou le corps du patient, et donc la distribution des hybrides htiARN.

L'agent d'imagerie peut notamment être utilisé dans les imageries de type :
- tomographie à émission de positons (Positron Emission Tomography (PET) en anglais) (l'agent d'imagerie pouvant être un composé comprenant un radionucléide, tel que ¹⁸F, ¹¹C, un chélate de cations métalliques ⁶⁸Ga, ⁶⁴Cu),
- tomographie d'émission monophotonique (TEMP) (Single photon émission computed tomography (SPECT) en anglais) (l'agent d'imagerie pouvant être un composé comprenant un radionucléide par exemple ¹²³I, ou un chélate de ^{99m}Tc ou ¹¹¹In),
- imagerie par résonance magnétique (IRM) (l'agent d'imagerie pouvant être un chélate de gadolinium ou un nanocristal magnétique tel qu'un nanocristal d'oxyde de fer, d'oxyde de manganèse ou de fer-platine FePt),
- imagerie optique ou imagerie aux rayons X (l'agent d'imagerie pouvant être un fluorophore lipophile ou un agent de contraste, par exemple une molécule iodée telle que l'iopamidol, l'amidotrizoate, ou des nanoparticules d'or).

De préférence, l'agent d'imagerie est un fluorophore lipophile permettant de réaliser de l'imagerie optique.

La nature du ou des fluorophores lipophiles utilisables n'est pas critique à partir du moment où ils sont compatibles avec l'imagerie in vivo (c'est à dire qu'ils sont biocompatibles et non toxiques). De préférence, les fluorophores utilisés comme agent d'imagerie absorbent et émettent dans le visible ou le proche infrarouge. Pour l'imagerie non invasive dans un tissu ou un organisme vivant (animal, homme) les fluorophores préférés absorbent et émettent dans le proche infrarouge. En effet, pour que la lumière d'excitation et la lumière émise par le fluorophore puissent mieux traverser les tissus, il convient d'utiliser des fluorophores absorbant et émettant dans le proche infrarouge, c'est-à-dire à une longueur d'onde comprise entre 640 et 900 nm.

A titre de fluorophore lipophile on peut par exemple citer les composés décrits dans le chapitre 13 ("Probes for Lipids and Membranes") du catalogue InVitrogen. De façon plus précise, on peut notamment citer, à titre de fluorophore, le vert d'indocyanine (ICG), les analogues d'acides gras et les phospholipides fonctionnalisés par un groupement fluorescent tels que les produits fluorescents vendus sous les dénominations commerciales Bodipy (R) tels que le Bodipy (R) 665/676 (Ex/Em.) ; les dérivés lipophiles de carbocyanines telles que le perchlorate de 1,1'--dioctadécyl-3,3,3',3'-tétraméthylindodicarbocyanine (DiD), par exemple vendu sous la référence D-307, le perchlorate de 3,3'-dihexadecyloxacarbocyanine (DiO), par exemple vendu sous la référence D1125, le perchlorate de 1,1'-dihexadecyl-3,3,3',3'-tetramethylindocarbocyanine (Dil), par exemple vendu sous la référence D384; les sondes fluorescentes dérivées de sphingolipides, de stéroïdes ou de lipopolysaccharides tels que les produits vendus sous les dénominations commerciales BODIPY ^{®} TR céramides, BODIPY ^{®} FL C5-lactosylcéramide, BODIPY ^{®} FL C5-ganglioside, BODIPY ^{®} FL cérébrosides ; les dérivés amphiphiles de cyanines, de rhodamines, de fluorescéines ou de coumarines tels que l'octadécyl rhodamine B, l'octadécyl ester de fluorescéine et la 4-heptadécyl-7- hydroxycoumarine ; et le diphénylhexatriène (DPH) et ses dérivés ; l'ensemble de ces produits étant vendus par la société Invitrogen.

Selon une forme de réalisation préférée de l'invention, le fluorophore est le vert d'indocyanine, le perchlorate de 1,1'--dioctadécyl-3,3,3',3'-tétraméthylindodicarbocyanine, le perchlorate de 3,3'-dihexadecyloxacarbocyanine, ou le perchlorate de 1,1'-dihexadecyl-3,3,3',3'-tetramethylindocarbocyanine.

### Agent thérapeutique

La nanoémulsion peut comporter un agent thérapeutique.

Les agents thérapeutiques susceptibles d'être encapsulés dans la nanoémulsion comprennent en particulier les principes actifs agissant par voie chimique, biologique ou physique. Ainsi, il peut s'agir de principes actifs pharmaceutiques ou d'agents biologiques tels que de l'ADN, des protéines, peptides ou anticorps ou encore des agents utiles pour des thérapies physiques tels que des composés utiles pour la thermothérapie, les composés relarguant de l'oxygène singulet lorsqu'ils sont excités par une lumière utiles pour la photothérapie et des agents radioactifs. De préférence, il s'agit de principes actifs administrés de façon parentérale.

Selon son affinité lipophile ou amphiphile, l'agent thérapeutique sera encapsulé par la phase dispersée ou se situera à l'interface des deux phases.

La nature des agents thérapeutiques encapsulés dans la nanoémulsion n'est pas particulièrement limitée. Cependant, la nanoémulsion est particulièrement intéressante pour des composés peu solubles, qui sont difficiles à formuler dans les systèmes d'administration classiques et pour les principes actifs utiles pour la photothérapie, dont le rendement quantique peut être préservé.

Du fait des conditions douces du procédé de préparation, la nanoémulsion décrite est particulièrement intéressante pour l'encapsulation d'agents thérapeutiques qui se dégradent à température élevée.

Parmi les principes actifs pharmaceutiques intéressants comme agents thérapeutiques, on peut citer en particulier les agents utilisés dans le traitement du SIDA, les agents utilisés dans le traitement des maladies cardiaques, les analgésiques, les anesthésiques, les anorexigènes, les anthelmintiques, les antiallergiques, les antiangineux, les antiarythmisants, les anticholinergiques, les anticoagulants, les antidépresseurs, les antidiabétiques, les antidiurétiques, les antiémétiques, les anticonvulsivants, les antifongiques, les antihistaminiques, les antihypertenseurs, les anti-inflammatoires, les anti-migraineux, les antimuscariniques, les antimycobactériens, les anticancéreux y compris les antiparkinsoniens, les antithyroïdiens, les antiviraux, les astringents, les agents bloquants, les produits sanguins, les substituts sanguins, les agents inotropes cardiaques, les agents cardiovasculaires, les agents du système nerveux central, les chélateurs, les agents de chimiothérapie, les facteurs de croissance hématopoïétiques, les corticostéroïdes, les antitussifs, les agents dermatologiques, les diurétiques, les dopaminergiques, les inhibiteurs de l'élastase, les agents endocrines, les alkaloïdes de l'ergot, les expectorants, les agents gastro-intestinaux, les agents génito-urinaires, le facteur de déclenchement de l'hormone de croissance, les hormones de croissance, les agents hématologiques, les agents hématopoïétiques, les hemostatiques, les hormones, les immunosuppresseurs, les interleukines, les analogues d'interleukines, les agents de régulation des lipides, la gonadolibérine, les myorelaxants, les antagonistes narcotiques, les nutriments, les agents nutritifs, les thérapies oncologiques, les nitrates organiques, les vagomimétiques, les prostaglandines, les antibiotiques, les agents rénaux, les agents respiratoires, les sédatifs, les hormones sexuelles, les stimulants, les sympathomimétiques, les anti-infectieux systémiques, le tacrolimus, les agents thrombolytiques, les agents thyroïdiens, les traitements pour les troubles de l'attention, les vasodilatateurs, les xanthines, les agents diminuant le cholestérol. Particulièrement visés sont les anticancéreux tels que le taxol (paclitaxel), la doxorubicine et le cisplatine.

Parmi les agents physiques ou chimiques, on peut citer notamment les isotopes radioactifs et les photo-sensibilisateurs.

Parmi les photo-sensibilisateurs, on peut citer notamment ceux appartenant à la classe des tétrapyrroles comme les porphyrines, les bactériochlorines, les phtalocyanines, les chlorines, les purpurines, les porphycènes, les phéophorbides, ou encore ceux appartenant à la classe des texaphyrines ou des hypericines. Parmi les photo-sensibilisateurs de première génération, on peut mentionner l'hémato-porphyrine et un mélange de dérivés d'hémato-porphyrine (HpD) (vendu sous la marque commerciale Photofrin^{®} par Axcan Pharma). Parmi les photo-sensibilisateurs de seconde génération, on peut mentionner le méta-tetra-hydroxyphenyl chlorine (mTHPC ; nom commercial Foscan^{®}, Biolitec AG) et le dérivé monoacide du cycle A de la benzoporphyrine (BPD-MA vendu sous la marque commerciale Visudyne^{®} par QLT et Novartis Opthalmics). Les formulations des photo-sensibilisateurs de seconde génération qui associent à ces photo-sensibilisateurs une molécule (lipide, peptide, sucre etc..) qualifiée de transporteur qui permet leur acheminement sélectif au niveau du tissu tumoral sont appelées photo-sensibilisateurs de troisième génération.

Parmi les agents biologiques, on peut mentionner les oligonucléotides, de l'ADN, de l'ARN, des siARN, les peptides et les protéines et les saccharides.

Bien entendu, l'agent thérapeutique peut être formulé directement sous sa forme active ou sous forme d'une pro-drogue. Par ailleurs, il est envisagé que plusieurs agents thérapeutiques puissent être formulés en association dans la nanoémulsion.

La quantité d'agent thérapeutique dépend de l'application visée ainsi que de la nature de l'agent. Toutefois, on cherchera généralement à formuler la nanoémulsion avec une concentration maximale en agent thérapeutique, notamment lorsqu'il s'agit d'agents thérapeutiques peu solubles, afin de limiter le volume et/ou la durée d'administration au patient.

Or il a été constaté que la présence du lipide solubilisant dans la phase dispersée permet d'incorporer une quantité importante de composés mêmes hydrophobes ou amphiphiles.

### Proportion de coeur dans les gouttelettes

Généralement, la proportion molaire des composants du coeur des gouttelettes par rapport aux composants des gouttelettes est de 10 à 80%, notamment de 25 à 75%, de préférence de 33,35 à 73,99% (ces pourcentages étant calculés sans prendre en compte l'hybride htiARN). Autrement dit, la proportion molaire (mol/mol) de l'ensemble (lipide solubilisant / éventuelle huile / éventuel agent d'imagerie / éventuel agent thérapeutique lipophile) par rapport à la phase dispersée (c'est-à-dire à tous les composants de la phase dispersée) est généralement de 10 à 80%, notamment de 25 à 75%, de préférence de 33,35 à 73,99%.

Généralement, la proportion massique (wt/wt) des composants du coeur des gouttelettes par rapport aux composants des gouttelettes est de 10 à 60%, notamment de 20 à 60%, de préférence de 23,53 à 59,51% (ces pourcentages étant calculés sans prendre en compte l'hybride htiARN). Autrement dit, la proportion massique de l'ensemble (lipide solubilisant / éventuelle huile / éventuel agent d'imagerie / éventuel agent thérapeutique lipophile) par rapport à la phase dispersée (c'est-à-dire à tous les composants de la phase dispersée) est généralement de 10 à 60%, notamment de 20 à 60%, de préférence de 23,53 à 59,51%.

Ces proportions molaires et/ou massiques sont particulièrement adaptées pour que la nanoémulsion avant complexation avec le htiARN soit stable au stockage, notamment qu'elle soit stable en étant stockée plus de 28 jours à 40°C, voire plus de 300 jours à 40°C. La stabilité peut notamment être mesurée en suivant la taille des gouttelettes, leur index de polydispersité et/ou leur potentiel zêta (par exemple par diffusion quasi-élastique de la lumière par un appareil de type ZetaSizer, Malvern).

### Phase aqueuse

La phase aqueuse de la nanoémulsion est de préférence constituée d'eau et/ou d'un tampon tel qu'un tampon phosphate comme par exemple du PBS ("Phosphate Buffer Saline") ou d'une solution saline, notamment de chlorure de sodium.

La phase aqueuse continue peut également comporter un agent épaississant tel que du glycérol, un saccharide, oligosaccharide ou polysaccharide, une gomme ou encore une protéine, de préférence du glycérol. En effet, l'utilisation d'une phase continue de viscosité plus élevée facilite l'émulsification et permet de ce fait de réduire le temps de sonication.

La phase aqueuse comporte avantageusement de 0 à 50% en poids, de préférence de 1 à 30% en poids et tout particulièrement de 5 à 20% en poids d'agent épaississant.

Bien entendu, la phase aqueuse peut contenir en outre d'autres additifs tels que des colorants, stabilisants et conservateurs en quantité appropriée.

La proportion de phase dispersée et de phase aqueuse est très variable. Cependant, le plus souvent, les nanoémulsions seront préparées avec 1 à 50%, de préférence 5 à 40% et tout particulièrement 10 à 30% en poids de phase dispersée et 50 à 99%, de préférence 60 à 95% et tout particulièrement 70 à 90 % en poids de phase aqueuse.

### Taille des gouttelettes

Les gouttelettes de la nanoémulsion avant complexation avec l'(les) hybride(s) htiARN (gouttelettes nues) ont généralement un diamètre compris entre 20 et 200 nm. Ce diamètre peut notamment être mesuré par diffusion quasi-élastique de la lumière sur un appareil ZetaSizer, Malvern.

Il est possible d'obtenir des gouttelettes de taille plus spécifique en adaptant les pourcentages des composants de la nanoémulsion.

Pour une nanoémulsion comprenant des gouttelettes non complexées à un hybride htiARN de taille comprise entre 20 et 40 nm, on préfèrera utiliser une nanoémulsion comprenant au moins 5% molaire de lipide amphiphile et :
- de 25 à 45% molaire de co-tensioactif (en dessous de 25% molaire, la nanoémulsion peut présenter des problèmes de stabilité), et/ou
- de 15 à 50% molaire de tensioactif cationique.

Pour une nanoémulsion comprenant des gouttelettes non complexées à un hybride htiARN de taille comprise entre 40 et 100 nm, on préfèrera utiliser une nanoémulsion comprenant au moins 5% molaire de lipide amphiphile et :
- de 45 à 50% molaire de co-tensioactif (en dessous de 45% molaire, la nanoémulsion peut présenter des problèmes de stabilité. Au-delà de 50%, l'efficacité en transfection de la nanoémulsion est moindre), et/ou
- de 30 à 40% molaire de tensioactif cationique. (en dessous de 30% molaire, l'efficacité en transfection de la nanoémulsion est moindre. Au-delà de 40%, la nanoémulsion peut présenter des problèmes de stabilité).

Pour une nanoémulsion comprenant des gouttelettes non complexées à un hybride htiARN de taille comprise entre 130 et 175 nm, on préfèrera utiliser une nanoémulsion comprenant au moins 5% molaire de lipide amphiphile et de 15 à 70% molaire d'au moins un tensioactif cationique et :
- de 10 à 25% molaire, notamment de 10 à 15% de co-tensioactif.

Les pourcentages molaires de lipide amphiphile, de tensioactif cationique et de co-tensioactif sont par rapport à l'ensemble (lipide amphiphile / tensioactif cationique / co-tensioactif / éventuel lipide fusogène).

Les gouttelettes du complexe hybride htiARN / gouttelettes (gouttelettes complexées à un hybride htiARN) ont généralement un diamètre compris entre 20 et 250 nm, typiquement entre 40 et 200 nm. Ce diamètre peut notamment être mesuré par diffusion quasi-élastique de la lumière sur un appareil ZetaSizer, Malvern.

### Localisation des composants dans le complexe

Les gouttelettes du complexe htiARN / gouttelette sous forme de nanoémulsion définie ci-dessus s'organisent sous forme de coeur - couronne, où :
- le coeur comprend :
   - le lipide solubilisant,
   - l'éventuelle huile,
   - l'éventuel agent d'imagerie,
   - l'éventuel agent thérapeutique lipophile,
- la couronne comprend :
   - le lipide amphiphile,
   - le tensioactif cationique,
   - le co-tensioactif (éventuellement greffé avec une molécule d'intérêt),
   - l'hybride htiARN,
   - l'éventuel lipide fusogène,
   - l'éventuel agent thérapeutique s'il est amphiphile.

### Procédé de préparation du complexe hybride htiARN / nanoparticule

Le complexe htiARN / nanoparticule sous forme de nanoémulsion définie ci-dessus peut être préparé en suivant le procédé de préparation décrit dans la demande FR 2 994 849, en utilisant le htiARN en tant que séquence nucléotidique susceptible de moduler des mécanismes endogènes d'ARN interférence.

Selon un troisième objet, l'invention concerne l'utilisation d'un hybride htiARN tel que défini ci-dessus pour stabiliser un complexe entre une nanoparticule et ledit ARN antisens.

L'invention concerne également une méthode de stabilisation d'un complexe d'un ARN antisens et d'une nanoparticule comprenant les étapes consistant à :
- fournir un ARN antisens, un premier brin d'ADN et un deuxième brin d'ADN tels que définis ci-dessus, et une nanoparticule, notamment telle que définie ci-dessus,
- hybrider dans un tampon d'hybridation deux équivalents molaire d'ARN antisens, un équivalent molaire de premier brin d'ADN et d'un équivalent molaire de deuxième brin d'ADN, ce par quoi un hybride htiARN tel que défini ci-dessus est obtenu,
- complexer l'hybride htiARN avec la nanoparticule.

Selon un quatrième objet, l'invention concerne une méthode d'introduction d'ARN antisens susceptible de moduler des mécanismes endogènes d'ARN interférence dans une cellule eucaryote, comprenant la mise en contact de la cellule eucaryote avec le complexe selon l'invention. Cette méthode est une méthode de transfection.

La méthode d'introduction peut être réalisée in vitro. Dans ce cas, généralement, la mise en contact de la cellule eucaryote avec le complexe selon l'invention a lieu dans une solution tampon, par exemple un milieu OptiMEM^{®}. La mise en contact dure généralement entre 8 et 96 heures, typiquement de l'ordre de 72 heures, à une température de l'ordre de 37°C.

Généralement, après l'étape de mise en contact, les cellules sont récupérées.

Selon un cinquième objet, l'invention concerne le complexe défini ci-dessus pour son utilisation pour la prévention ou le traitement d'une maladie, en particulier une maladie du système digestif, telle qu'une maladie chronique inflammatoire de l'intestin, notamment la maladie de Crohn ou la rectocolite hémorragique.

L'invention concerne également une méthode de prévention ou de traitement d'une maladie, en particulier d'une maladie du système digestif, telle qu'une maladie chronique inflammatoire de l'intestin, notamment la maladie de Crohn ou la rectocolite hémorragique comprenant l'administration à un mammifère en ayant besoin, de préférence un humain en ayant besoin, d'une quantité efficace du complexe défini ci-dessus.

Le complexe hybride htiARN / nanoparticule sous forme de nanoémulsion peut comprendre un agent thérapeutique permettant de traiter la maladie, ce qui permet de bénéficier d'un double effet thérapeutique : celui induit par l'hybride htiARN qui permet de moduler des mécanismes endogènes d'ARN interférence et celui induit par l'agent thérapeutique.

L'administration peut être parentérale, intraveineuse, orale, topique, intra rectale ou ophtalmologique, et est de préférence orale, ce qui est possible grâce à la stabilité du complexe selon l'invention aux fluides physiologiques complexes et notamment aux milieux intestinaux.

L'invention est illustrée au vu des figures et exemples qui suivent.

### Figures

[Fig 1] Figure 1 : Structure de l'hybride htiARN
[Fig 2] Figure 2 : Structure des deux unités monomériques de l'hybride htiARN vu comme un dimère
[Fig 3] Figure 3 : Structure d'un exemple d'hybride htiARN (« htiGFP ») selon l'invention [Fig 4] Figure 4 : Structure d'une unité monomérique de l'hybride htiGFP (comparatif).
[Fig 5] Figure 5 : Structure d'un dimère premier brin d'ADN / deuxième brin d'ADN de d'hybride htiGFP, les deux brins étant hybridés par S1 et S2 (comparatif).
[Fig. 6] Figure 6 : Structure d'un hybride htiARN contrôle (« htiCTRL »), dont l'ARN antisens de séquence SEQ ID NO:7 n'est pas susceptible de moduler les mécanismes endogènes d'ARN interférence (contrôle).

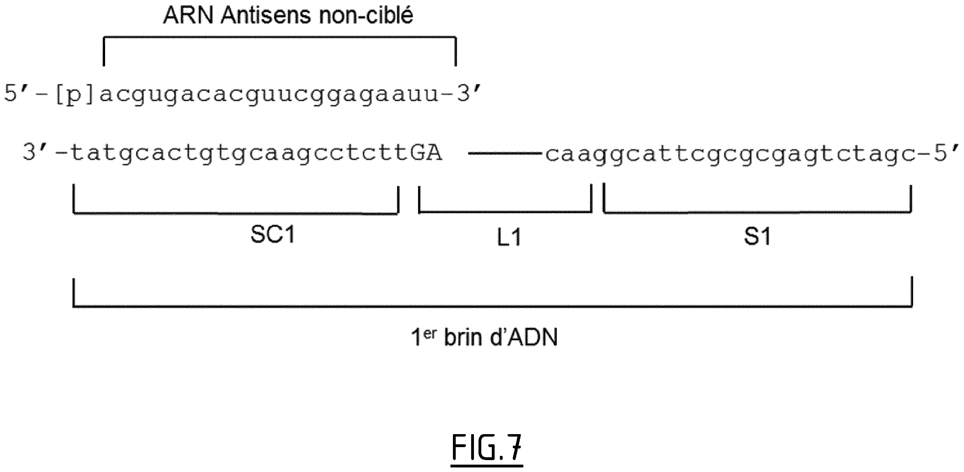

[Fig. 7] : Structure d'une unité monomérique de l'hybride htiCTRL

### Exemples

### Exemple 1 : Hybride htiGFP

### 1.1. Préparation de l'hybride htiGFP

L'hybridation des deux unités monomériques en un dimère ([Fig 2] figure 2) a été effectuée par chauffage, à l'aide du protocole indiqué au tableau 2, afin de former l'hybride htiGFP de structure détaillée à [Fig 3] la figure 3. Un ARN interférant ciblant l'ARN messager du gène codant pour la GFP a été utilisé comme ARN antisens.

### [Tableau 2]

**Tableau 2 : Protocole de préparation d'une structure hybride ADN-ARN « htiGFP » « susceptible de moduler des mécanismes endogènes d'ARN interférence**

| Oligonucléotide | séquence | fournisseur | Equivalent molaire | Tampon d'hybridation | Protocole |
|---|---|---|---|---|---|
| ARN antisens | SEQ ID NO:3 | IDT DNA | 2 | 100mM KCl | Chauffer : |
| | | | | 1mM MgCl₂ | 1) 5 minutes à 90°C |
| 1^{er} brin d'ADN | SEQ ID NO:1 | IDT DNA | 1 | 30mM | |
| | | | | HEPES | 2) 5 minutes à 80°C |
| 2^{ème} brin d'ADN | SEQ ID NO:2 | IDT DNA | 1 | pH7,3 | |
| | | | | | 3) 20 minutes à 70°C Laisser refroidir lentement jusqu'à 25°C |

L'hybridation effective des deux unités monomériques en un dimère hybride htiGFP a été contrôlée par une électrophorèse sur gel d'agarose (E-gel EX 4% Agarose Sybr Gold, Invitrogen). Le temps de migration était de 15 minutes. Un témoin de poids moléculaire (GeneRuler 50 bp DNA Ladder, Thermo Scientific) a été utilisé pour démontrer que la taille de la structure obtenue se situe entre les marqueurs 100 paires de bases (100 pb) et 50 paires de bases (50 pb). La structure de l'hybride htiGFP est de 69 paires de bases (n_{SC1} + n_{L1} + n_{S1} + n_{ARN} = n_{SC2} + n_{L2} + n_{S2} + n_{ARN} = 69).

### 1.2. Préparation de structures comparatives

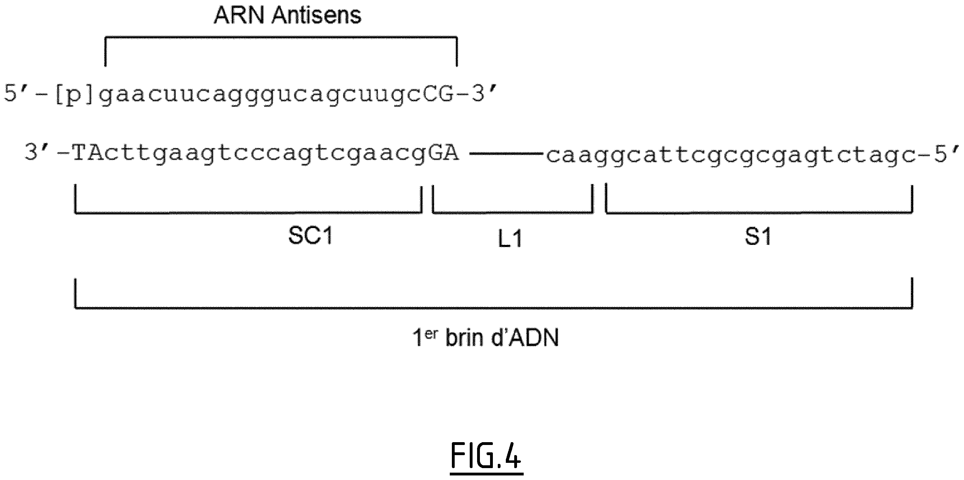

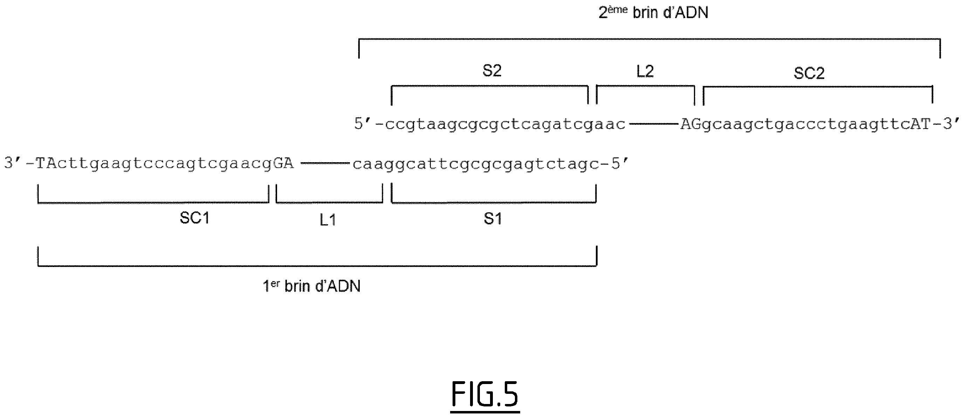

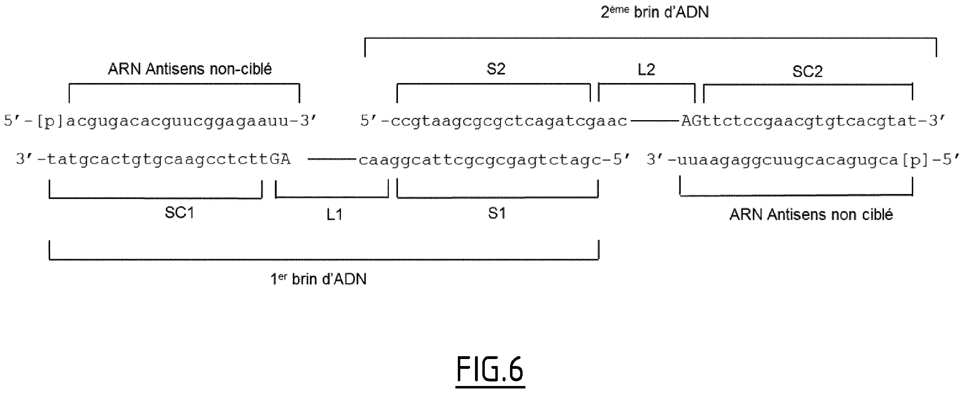

Par ailleurs, pour la réalisation d'exemples comparatifs ont été préparés :
- une unité monomérique de l'hybride htiGFP ([Fig 4] figure 4).
- un dimère constitué du premier brin d'ADN et du deuxième brin d'ADN, les deux brins étant hybridés par S1 et S2 (dimère exempt d'ARN antisens) ([Fig 5] figure 5).
- un autre hybride htiCTRL contrôle, dont l'ARN antisens n'est pas susceptible de moduler les mécanismes endogènes d'ARN interférence ([Fig 6] figure 6). Cet ARN antisens non ciblé de séquence SEQ ID NO:7 (Kim et al., 2005) n'a pas de cible spécifique sur le génome humain et est utilisé pour contrôle. Le premier brin d'ADN de l'hybride htiCTRL a la séquence SEQ ID NO:8 et le deuxième brin a la séquence SEQ ID NO:9. Les ARN antisens et SC1 et SC2 diffèrent entre htiGFP et htiCTRL. Par contre, les bras espaceurs L1 et L2 et les séquences nucléotidiques S1 et S2 de l'hybride htiCTRL sont identiques à ceux de l'hybride htiGFP.

### 1.3. Activité fonctionnelle de l'hybride htiGFP

Une lignée de cellules tumorales de cancer de la prostate surexprimant le gène codant pour la Green Fluorescent Protein (PC3-GFP) a été utilisée pour valider l'activité fonctionnelle d'ARN interférence des différentes structures décrites en 1.1 et 1.2.

Chaque ARN interférent a été transfecté dans la lignée cellulaire PC3-GFP à une concentration finale de 5 à 20 nM à l'aide du réactif Lipofectamine RNAimax, suivant les recommandations du fabricant.

La fluorescence à 510 nm (longueur d'onde d'émission de la GFP) de chaque cellule a été quantifiée par microscopie confocale (>300 cellules par conditions). Chaque condition a été reproduite à N = 3 expériences indépendantes, +/- écart-type standard. Les résultats sont fournis au tableau 3.

### [Tableau 3]

**Tableau 3 : Intensité de fluorescence de la GFP par la cellule en fonction de la structure utilisée**

| | Intensité de fluorescence de la GFP par cellule (Unité Arbitraire, ± écart-type standard, N=3 expériences indépendantes) | | |
|---|---|---|---|
| concentration | 5nM | 10nM | 20nM |
| Non traité | 441,23 ± 41,46 | 474,68 ± 103,75 | 462,71 ± 57,27 |
| ARN antisens non ciblé siAllStar (Qiagen et son brin complémentaire) (comp.) | 479,02 ± 26,48 | 529,92 ± 86,86 | 561,02 ± 31,85 |
| ARN antisens GFP siGFP (SEQ ID NO:3 et son brin complémentaire) (comp.) | 147,38 ± 13,94 | 141,95 ± 21,24 | 117,33 ± 27,66 |
| unité monomérique de l'hybride htiCTRL ([Fig 7] figure 7) (comp.) | 456,09 ± 24,14 | 458,12 ± 48,42 | 540,6 ± 84,28 |
| unité monomérique de l'hybride htiGFP ([Fig 4] figure 4) (comp.) | 256,43 ± 20,37 | 245,89 ± 16,42 | 149,55 ± 29,65 |
| dimère 1^{er} brin d'ADN / 2^{ème} brin d'ADN de d'hybride htiGFP ([Fig 5] figure 5) (comp.) | 523,38 ± 69,67 | 567,72 ± 57,99 | 519,98 ± 67,82 |
| htiCTRL ([Fig 6] figure 6) (comp.) | 502,53 ± 38,22 | 519,1 ± 67,32 | 528,58 ± 46,05 |
| htiGFP ([Fig 3] figure 3) (invention) | 311,79 ± 11,7 | 309,41 ± 75,99 | 237,15 ± 15,5 |

L'activité de l'hybride htiGFP, conserve une activité similaire d'ARN interférence comparé à un siARN dont la séquence antisens SEQ ID NO:3 est identique. En effet, d'après le tableau 3, le siARN ciblant le gène codant pour la GFP (siGFP) diminue fortement l'expression de son gène cible, visualisé par une diminution de l'intensité de la GFP par cellule, lorsque les cellules PC3-GFP sont transfectées par ce siARN. Après l'intégration de la même séquence d'ARN antisens au sein d'une structure hybride ADN/ARN en tandem inversé (htiGFP), tout comme sa sous-unité monomérique (unité monomérique de l'hybride htiGFP) on observe toujours une activité similaire d'ARN interférence (tableau 3). Cette activité est cependant un peu moins importante qu'avec un siARN composé uniquement d'ARN (siGFP).

### Exemple 2 : Préparation d'un complexe nanoparticule lipidique / hybride htiARN

### 2.1. Préparation de nanoparticules lipidiques

Les nanoparticules lipidiques (LNP) sont préparées par mélange des phases organiques et aqueuses par un procédé ultrasonique, permettant de générer des nano-gouttelettes. Après homogénéisation à 55°C, les deux phases sont mélangées et des cycles de sonication sont réalisés à 55°C pendant 5 minutes (alternant 10 secondes de sonication et 30 secondes de repos). Un sonicateur à sonde conique de 3 mm est utilisé (AV505 Ultrasonic processor, Sonics), réglé sur une puissance de 45%.
Les composants non-encapsulés sont séparés des LNPs par dialyse dans un volume de tampon PBS (Phosphate-Buffered Saline) équivalent à 200 fois le volume des LNPs. Le tampon PBS est changé deux fois au cours de la dialyse, d'une durée totale de 24h. Après caractérisation, les LNPs sont filtrées sur une membrane cellulose d'une porosité de 0.22 µm.

Le diamètre hydrodynamique, l'index de polydispersité (PDI) et le potentiel zêta des nanoparticules lipidiques sont mesurés à l'aide d'un instrument Zeta Sizer Nano (NanoZS, Malvern). Le diamètre hydrodynamique et l'index de polydispersité sont mesurés à une concentration de LNP de 0.6 mg/mL dans un tampon PBS à 25°C. Le potentiel zeta est mesuré à une concentration de 0.4 mg/mL dans un tampon 1 mM NaCl, pH 7,4 à 25°C.

### [Tableau 4]

**Tableau 4: Composition des émulsions comprenant les nanoparticules CL40 et CL80**

| | | Phase organique | | | | | Phase aqueuse | | |
|---|---|---|---|---|---|---|---|---|---|
| | | LIPOID S PC-3 | DOTAP | DOPE | SUPER REFINED^{®} SOYBEAN USP EP-LQ-(MH) | SUPPOCIRE STANDARD | MYRJ^{™} S40-PW-(MV) | PBS | Glycérol |
| CL40 | % (Phase solide) | 3,43 | 30,4 | 3,61 | 18,24 | 6,11 | 38,24 | - | - |
| | mg | 3,7 | 32,8 | 3,9 | 19,7 | 6,6 | 41,3 | 1480 | 800 |
| CL80 | % (Phase solide) | 1,47 | 10,01 | - | 44,88 | 14,95 | 28,77 | - | - |
| | mg | 2,2 | 15 | - | 67,1 | 22,4 | 43,1 | 1480 | 800 |

### [Tableau 5]

**Tableau 5 : Listes des produits utilisés**

| Nom commercial | Fournisseur | No CAS: | Description |
|---|---|---|---|
| SUPPOCIRE STANDARD | Gattefossé | 85665-33-4 | Glycerides, C10-18; Triglycerides C10-C18 |
| MYRJ^{™} S40-PW-(MV) | CRODA | 9004-99-3 | Polyoxyethylene fatty acid ester |
| SUPER REFINED^{®} SOYBEAN USP EP-LQ-(MH) | CRODA | 232-274-4 | Soybean oil |
| LIPOID S PC-3 | Lipoid | 97281-48-6 | Phosphatidylcholine hydrogenated (phospholipids) |
| DOTAP | MERCK | 132172-61-3 | 1,2-dioleoyl-3-trimethylammonium-propane |
| DOPE | AvantiPolar | 4004-05-1 | 1,2-dioleoyl-sn-glycero-3-phosphoethanolamine |
| Phosphate Buffered Saline | Sigma | - | Tampon à pH neutre |
| Glycérol | Sigma | 56-81-5 | - |

### 2.2. Préparation d'un complexe nanoparticule lipidique / hybride htiARN

L'hybride htiARN de l'exemple 1 a été complexé aux nanoparticules préparées à l'exemple 3.1.

Les complexes ont été formés entre les différents acides nucléiques et les nanoparticules lipidiques pendant 20 minutes dans un tampon de réaction (154 mM NaCl, 10 mM HEPES, pH 7.2). Les quantités de nanoparticules lipidiques sont ajustées pour maintenir un ratio N/P constant avec N/P = 36 (N : groupement amine DOTAP/DOPE, P : groupement phosphate de l'acide nucléique correspondant). Une quantité de milieu artificiel SHIME (Prodigest) est ajoutée pour une concentration finale allant de 0 à 90% de milieu SHIME (% V/V). Les résultats sont fournis au tableau 6.

### [Tableau 6]

**Tableau 6 : Stabilité selon la proportion de milieu SHIME**

| | Volume LNP (µL) | Volume siAllStar 20µM (µL) | Volume htiGFP 20µM (µL) | Tampon 154 mM NaCl, 10 mM Hepes, pH7.2 (µL) | Milieu SHIME (µL) | Volume total (µL) | Ratio N/P | % Milieu SHIME | % de Stabilité (Ratio du contrôle SHIME + ARN interférent) |
|---|---|---|---|---|---|---|---|---|---|
| 1 | 0 | 1 | | 39,0 | 0 | 40,00 | 36 | 0,0% | - |
| 2 | 0 | 1 | | 19,0 | 20 | 40,00 | 36 | 50,0% | - |
| 3 | 0,7 | 1 | | 38,3 | 0 | 40,00 | 36 | 0,0% | 92,85 |
| 4 | 0,7 | 1 | | 18,3 | 20 | 40,00 | 36 | 50,0% | 46,11 |
| 5 | 0,7 | 1 | | 14,3 | 24 | 40,00 | 36 | 60,0% | 20,48 |
| 6 | 0,7 | 1 | | 10,3 | 28 | 40,00 | 36 | 70,0% | 2,093 |
| 7 | 0,7 | 1 | | 6,3 | 32 | 40,00 | 36 | 80,0% | 5,114 |
| 8 | 0,7 | 1 | | 2,3 | 36 | 40,00 | 36 | 90,0% | 3,368 |
| 9 | 0 | | 1 | 39,0 | 0 | 40,00 | 36 | 0,0% | - |
| 10 | 0 | | 1 | 19,0 | 20 | 40,00 | 36 | 50,0% | - |
| 11 | 2 | | 1 | 37,0 | 0 | 40,00 | 36 | 0,0% | 96,78 |
| 12 | 2 | | 1 | 17,0 | 20 | 40,00 | 36 | 50,0% | 95,62 |
| 13 | 2 | | 1 | 13,0 | 24 | 40,00 | 36 | 60,0% | 95,82 |
| 14 | 2 | | 1 | 9,0 | 28 | 40,00 | 36 | 70,0% | 94,51 |
| 15 | 2 | | 1 | 5,0 | 32 | 40,00 | 36 | 80,0% | 93,13 |
| 16 | 2 | | 1 | 1,0 | 36 | 40,00 | 36 | 90,0% | 92,89 |

### 2.3. Augmentation de la stabilité du complexe nanoparticule / htiGFP par rapport à un complexe nanoparticule / siGFP dans un fluide biologique complexe

La stabilité :
- du complexe nanoparticule / siARN contrôle (siAllstar, Qiagen, 21 paires de bases,) ou
- du complexe nanoparticule / htiGFP
a été suivie par électrophorèse sur gel d'agarose (E-gel 2% Agarose Bromure d'Ethidium, Invitrogen, temps de migration 10 minutes) après exposition des complexes à des concentrations croissantes de milieu artificiel SHIME (Prodigest) pour reproduire les conditions physiologiques au niveau du colon distal (100% SHIME).

Lorsque l'on augmente la concentration en milieu SHIME, une décomplexation des complexes nanoparticule / siAlIStar à partir d'une concentration de 50% en milieu SHIME est observable car une migration du siAlIStar est observée sur gel d'agarose. En revanche, grâce à une interaction électrostatique plus importante, le complexe nanoparticule / htiGFP reste stable dans ce fluide biologique comme l'indique l'absence de migration du htiGFP sur gel d'agarose.

## Revendications

1. Molécule hybride ADN/ARN, appelée hybride htiARN, comprenant :
- deux brins d'ARN antisens de séquences identiques et étant susceptibles de moduler des mécanismes endogènes d'ARN interférence, chacun comprenant un nombre n_{ARN} de nucléotides de 18 à 30, et ayant une extrémité 5' phosphorylée,
- un premier brin d'ADN dont la séquence nucléotidique comprend une séquence nucléotidique S1 liée par son extrémité 3' à l'extrémité 5' d'un bras espaceur nucléotidique L1 lié par son extrémité 3' à l'extrémité 5' d'une séquence nucléotidique SC1, et
- un deuxième brin d'ADN dont la séquence nucléotidique comprend une séquence nucléotidique S2 liée par son extrémité 3' à l'extrémité 5' d'un bras espaceur nucléotidique L2 lié par son extrémité 3' à l'extrémité 5' d'une séquence nucléotidique SC2,
où :
- la séquence nucléotidique SC1 comprend un nombre n_{SC1} nucléotides avec n_{SC1} de (n_{ARN} - 3) à (n_{ARN} + 3), et présente au moins 40 % d'identité de séquence, déterminé par une méthode d'alignement globale, avec la séquence complémentaire de l'ARN antisens, de sorte que la séquence nucléotidique SC1 est hybridée par complémentarité à un premier des deux brins d'ARN antisens,
- la séquence nucléotidique SC2 comprend un nombre n_{SC2} nucléotides avec n_{SC2} de (n_{ARN} - 3) à (n_{ARN} + 3), et présente au moins 40 % d'identité de séquence, déterminé par une méthode d'alignement globale, avec la séquence complémentaire de l'ARN antisens, de sorte que la séquence nucléotidique SC2 est hybridée par complémentarité au deuxième des deux brins d'ARN antisens,
- la séquence nucléotidique S1 du premier brin d'ADN est complémentaire de la séquence nucléotidique S2 du deuxième brin d'ADN, de sorte que la séquence nucléotidique S1 du premier brin d'ADN est hybridée par complémentarité à la séquence nucléotidique S2 du deuxième brin d'ADN,
- les deux séquences nucléotidiques S1 et S2 ont un même nombre n_{S1-S2} de nucléotides, n_{S1-S2} étant un nombre de 16 à 30,
- le bras espaceur nucléotidique L1 du premier brin d'ADN comprend un nombre n_{L1} de nucléotides et le bras espaceur nucléotidique L2 du deuxième brin d'ADN comprend un nombre n_{L2} de nucléotides, n_{L1} et n_{L2} représentant indépendamment un nombre de 1 à 15,
- les deux brins d'ARN antisens, le premier brin d'ADN et/ou le deuxième brin d'ADN sont éventuellement porteurs d'un ou plusieurs groupes lipidiques.

2. Hybride htiARN selon la revendication 1 dans lequel :
- n_{S1-S2} est un nombre entier de 18 à 22, et/ou
- nu et n_{L2} sont indépendamment un nombre entier de 1 à 15, notamment de 2 à 10, et/ou
- la somme n_{SC1} + n_{L1} + n_{S1} + n_{ARN} et/ou la somme n_{SC2} + n_{L2} + n_{S2} + n_{ARN} est(sont) supérieure(s) ou égale(s) à 60.

3. Complexe d'au moins une nanoparticule et d'au moins un hybride htiARN selon la revendication 1 ou 2.

4. Complexe selon la revendication 3, dans lequel la nanoparticule est cationique.

5. Complexe selon la revendication 3 ou 4, dans lequel la nanoparticule est une nanoparticule inorganique, organique ou hybride organique/inorganique.

6. Complexe selon l'une quelconque des revendications 3 à 5, dans lequel la nanoparticule est une nanoparticule organique cationique comprenant au moins un lipide, telle qu'un liposome ou une gouttelette d'une nanoémulsion comprenant une phase aqueuse continue et au moins une phase lipidique dispersée.

7. Complexe selon l'une quelconque des revendications 3 à 6, sous forme d'une nanoémulsion comprenant une phase aqueuse continue et au moins une phase dispersée qui comprend :
a) au moins 5% molaire de lipide amphiphile,
b) de 15 à 70% molaire d'au moins un tensioactif cationique comprenant :
i) au moins un groupe lipophile choisi parmi :
- un groupe R ou R-(C=O)-, où R représente une chaîne hydrocarbonée linéaire comprenant de 11 à 23 atomes de carbone,
- un ester ou un amide d'acides gras comprenant de 12 à 24 atomes de carbone et de phosphatidyléthanolamine, et
- un poly(oxyde de propylène), et
ii) au moins un groupe hydrophile comprenant au moins un groupe cationique choisi parmi :
- un groupe alkyle linéaire ou ramifié comprenant de 1 à 12 atomes de carbone et interrompu et/ou substitué par au moins un groupe cationique, et
- un groupe polymérique hydrophile comprenant au moins un groupe cationique, et
c) de 10% à 55% molaire d'un co-tensioactif comprenant au moins une chaîne poly(oxyde d'éthylène) comprenant au moins 25 unités d'oxyde d'éthylène,
d) un lipide solubilisant comprenant au moins un glycéride d'acides gras,
e) éventuellement un lipide fusogène,
f) un hybride htiARN selon la revendication 1 ou 2,
où les pourcentages molaires de lipide amphiphile, de tensioactif cationique et de co-tensioactif sont par rapport à l'ensemble (lipide amphiphile / tensioactif cationique / co-tensioactif / éventuel lipide fusogène).

8. Utilisation d'un hybride htiARN selon la revendication 1 ou 2 pour stabiliser un complexe entre ledit ARN antisens et une nanoparticule.

9. Méthode d'introduction *in vitro* d'un ARN antisens susceptible de moduler des mécanismes endogènes d'ARN interférence dans une cellule eucaryote, comprenant la mise en contact de la cellule eucaryote avec le complexe selon l'une quelconque des revendications 3 à 7.

10. Complexe selon l'une quelconque des revendications 3 à 7 pour son utilisation pour la prévention ou le traitement d'une maladie, en particulier une maladie du système digestif, telle qu'une maladie chronique inflammatoire de l'intestin, notamment la maladie de Crohn ou la rectocolite hémorragique.

## Patentansprüche

1. Hybrides DNA/RNA-Molekül, das als hybride htiRNA bezeichnet wird, umfassend:
- zwei Antisense-RNA-Stränge, die identische Sequenzen aufweisen und geeignet sind, endogene RNA-Interferenzmechanismen zu modulieren, jeweils umfassend eine Nukleotidanzahl n_{RNA} von 18 bis 30, und die ein phosphoryliertes 5'-Ende aufweisent,
- einen ersten DNA-Strang, dessen Nukleotidsequenz eine Nukleotidsequenz S1 umfasst, die an ihrem 3'-Ende an das 5'-Ende eines Nukleotid-Spacerarm L1 gebunden ist, der an seinem 3'-Ende an das 5'-Ende einer Nukleotidsequenz SC1 gebunden ist, und
- einen zweiten DNA-Strang, dessen Nukleotidsequenz eine Nukleotidsequenz S2 umfasst, die an ihrem 3'-Ende an das 5'-Ende eines Nukleotid-Spacerarm L2 gebunden ist, der an seinem 3'-Ende an das 5'-Ende einer Nukleotidsequenz SC2 gebunden ist,
wobei:
- die Nukleotidsequenz SC1 eine Anzahl von n_{SC1} Nukleotiden umfasst, wobei n_{SC1} von (n_{RNA} - 3) bis (n_{RNA} + 3) und mindestens 40 % Sequenzidentität, bestimmt durch ein globales Alignment-Verfahren, mit der komplementären Sequenz der Antisense-RNA aufweist, sodass die Nukleotidsequenz SC1 durch Komplementarität an einen ersten der zwei Antisense-RNA-Stränge hybridisiert ist,
- die Nukleotidsequenz SC2 eine Anzahl von n_{SC2} Nukleotiden umfasst, wobei n_{SC2} von (n_{RNA} - 3) bis (n_{RNA} + 3) und mindestens 40 % Sequenzidentität, bestimmt durch ein globales Alignment-Verfahren, mit der komplementären Sequenz der Antisense-RNA aufweist, sodass die Nukleotidsequenz SC2 durch Komplementarität an einen zweiten der zwei Antisense-RNA-Stränge hybridisiert ist,
- die Nukleotidsequenz S1 des ersten DNA-Strangs komplementär zu der Nukleotidsequenz S2 des zweiten DNA-Strangs ist, sodass die Nukleotidsequenz S1 des ersten DNA-Strangs durch Komplementarität an die Nukleotidsequenz S2 des zweiten DNA-Strangs hybridisiert ist,
- die zwei Nukleotidsequenzen S1 und S2 die gleiche Anzahl n_{S1-S2} an Nukleotiden aufweisen, wobei n_{S1-S2} eine Zahl von 16 bis 30 ist,
- der Nukleotid-Spacerarm L1 des ersten DNA-Strangs eine Anzahl von n_{L1} Nukleotiden umfasst und der Nukleotid-Sspacerarm L2 des zweiten DNA-Strangs eine Anzahl von n_{L2} Nukleotiden umfasst, wobei n_{L1} und n_{L2} unabhängig eine Zahl von 1 bis 15 darstellen,
- die zwei Antisense-RNA-Stränge, der erste DNA-Strang und/oder der zweite DNA-Strang optional eine oder mehrere Lipidgruppen tragen.

2. Hybride htiRNA nach Anspruch 1, wobei:
- n_{S1-S2} eine ganze Zahl von 18 bis 22 ist, und/oder
- n_{L1} und n_{L2} unabhängig eine ganze Zahl von 1 bis 15, insbesondere von 2 bis 10, sind und/oder
- die Summe n_{SC1} + n_{L1} + n_{S1} + n_{RNA} und/oder die Summe n_{SC2} + n_{L2} + n_{S2} + n_{RNA} größer als oder gleich wie 60 ist/sind.

3. Komplex aus mindestens einem Nanopartikel und mindestens einer hybriden htiRNA nach Anspruch 1 oder 2.

4. Komplex nach Anspruch 3, wobei das Nanopartikel kationisch ist.

5. Komplex nach Anspruch 3 oder 4, wobei das Nanopartikel ein anorganisches, organisches oder hybrides organisch/anorganisches Nanopartikel ist.

6. Komplex nach einem der Ansprüche 3 bis 5, wobei das Nanopartikel ein kationisches organisches Nanopartikel ist, umfassend mindestens ein Lipid umfasst, wie beispielsweise ein Liposom oder ein Tröpfchen einer Nanoemulsion, umfassend eine kontinuierliche wässrige Phase und mindestens eine dispergierte Lipidphase.

7. Komplex nach einem der Ansprüche 3 bis 6 in Form einer Nanoemulsion, umfassend eine kontinuierliche wässrige Phase und mindestens eine dispergierte Phase, die Folgendes umfasst:
a) mindestens 5 Mol-% amphiphiles Lipid,
b) 15 bis 70 Mol-% mindestens eines kationischen Tensids, umfassend:
i) mindestens eine lipophile Gruppe, ausgewählt aus:
- einer Gruppe R oder R-(C=O)-, wobei R eine lineare Kohlenwasserstoffkette umfassend 11 bis 23 Kohlenstoffatome darstellt,
- einen Ester oder ein Fettsäureamid umfassend 12 bis 24 Kohlenstoffatome und Phosphatidylethanolamin, und
- ein Poly(propylenoxid), und
ii) mindestens eine hydrophile Gruppe umfassend mindestens eine kationische Gruppe, die ausgewählt ist aus:
- einer linearen oder verzweigten Alkylgruppe umfassend 1 bis 12 Kohlenstoffatome und unterbrochen und/oder substituiert durch mindestens eine kationische Gruppe, und
- einer hydrophilen polymeren Gruppe umfassend mindestens eine kationische Gruppe, und
c) von 10 Mol-% bis 55 Mol-% eines Co-Tensids umfassend mindestens eine Polyethylenoxidkette umfassend mindestens 25 Ethylenoxideinheiten,
d) einem solubilisierenden Lipid umfassend mindestens ein Fettsäureglycerid,
e) optional einem fusogenen Lipid,
f) einer hybriden htiRNA nach Anspruch 1 oder 2,
wobei sich die molaren Prozentsätze des amphiphilen Lipids, des kationischen Tensids und des Co-Tensids auf die Gesamtheit (amphiphiles Lipid/kationisches Tensid/Co-Tensid/optional fusogenes Lipid) beziehen.

8. Verwendung einer hybriden htiRNA nach Anspruch 1 oder 2 zum Stabilisieren eines Komplexes zwischen der Antisense-RNA und einem Nanopartikel.

9. Verfahren zur in vitro-Einführung einer Antisense-RNA, die endogene RNA-Interferenzmechanismen modulieren kann, in eine eukaryotische Zelle, umfassend ein Inkontaktbringen der eukaryotischen Zelle mit dem Komplex nach einem der Ansprüche 3 bis 7.

10. Komplex nach einem der Ansprüche 3 bis 7 für seine Verwendung zur Vorbeugung oder Behandlung einer Krankheit, insbesondere einer Krankheit des Verdauungssystems, wie beispielsweise einer chronisch-entzündlichen Darmerkrankung, insbesondere Morbus Crohn oder Colitis ulcerosa.

## Claims

1. A hybrid DNA/RNA molecule, called htiRNA hybrid, comprising:
- two antisense RNA strands of same sequences and able to modulate endogenous mechanisms of RNA interference, each comprising a number of nucleotides n_{RNA} from 18 to 30, and having a phosphorylated 5' end;
- a first DNA strand, having a nucleotide sequence comprising a nucleotide sequence S1 linked via its 3' end to the 5' end of a nucleotide spacer arm L1 linked by its 3' end to the 5' end of a nucleotide sequence SC1, and
- a second DNA strand, having a nucleotide sequence comprising a nucleotide sequence S2 linked via its 3' end to the 5' end of a nucleotide spacer arm L2 inked via its 3' end to the 5' end of a nucleotide sequence SC2,
wherein:
- the nucleotide sequence SC1 comprises a number n_{SC1} of nucleotides with n_{SC1} from (n_{RNA} - 3) to (n_{RNA}+ 3), and has at least 40 % sequence identity, determined by a global alignment method, with the complementary sequence of the antisense RNA, so that the nucleotide sequence SC1 is hybridized via complementarity to a first of the two antisense RNA strands;
- the nucleotide sequence SC2 comprises a number n_{SC2} of nucleotides with n_{SC2} from (n_{RNA} - 3) to (n_{RNA} + 3), and has at least 40 % sequence identity, determined by a global alignment method, with the complementary sequence of the antisense RNA, so that the nucleotide sequence SC2 is hybridized via complementarity to the second of the two antisense RNA strands;
- the nucleotide sequence S1 of the first DNA strand is complementary to the nucleotide sequence S2 of the second DNA strand, so that the nucleotide sequence S1 of the first DNA strand is hybridized via complementarity to the nucleotide sequence S2 of the second DNA strand;
- the two nucleotide sequences S1 and S2 have the same number n_{S1-S2} of nucleotides, n_{S1-S2} being a number of 16 to 30;
- the nucleotide spacer arm L1 of the first DNA strand comprises a number n_{L1} of nucleotides and the nucleotide spacer arm L2 of the second DNA strand comprises a number n_{L2} of nucleotides, n_{L1} and n_{L2} independently being a number of 1 to 15;
- the two antisense RNA strands, the first DNA strand and/or the second DNA strand optionally carry one or more lipid groups.

2. The htiRNA hybrid according to claim 1, wherein:
- n_{S1-S2} is an integer of 18 to 22; and/or
- n_{L1} and n_{L2} are independently an integer of 1 to 15, in particular of 2 to 10; and/or
- the sum n_{SC1} + n_{L1} + n_{S1} + n_{RNA} and/or the sum n_{SC2} + n_{L2} + n_{S2}+ n_{RNA} are higher than or equal to 60.

3. A complex of at least one nanoparticle and at least one htiRNA hybrid according to claim 1 or 2.

4. The complex according to claim 3, wherein the nanoparticle is cationic.

5. The complex according to claim 3 or 4, wherein the nanoparticle is an inorganic, organic or hybrid organic/inorganic nanoparticle.

6. The complex according to any of claims 3 to 5, wherein the nanoparticle is a cationic organic nanoparticle comprising at least one lipid such as a liposome or droplet of a nanoemulsion comprising a continuous aqueous phase and at least one dispersed lipid phase.

7. The complex according to any of claims 3 to 6, in the form of a nanoemulsion comprising a continuous aqueous phase and at least one dispersed phase which comprises:
a) at least 5 mole % of amphiphilic lipid;
b) 15 to 70 mole % of at least one cationic surfactant comprising:
i) at least one lipophilic group selected from among:
- a group R or R-(C=O)-, where R is a linear hydrocarbon chain having 11 to 23 carbon atoms,
- a fatty acid ester or amide comprising 12 to 24 carbon atoms and phosphatidylethanolamine, and
- a polypropylene oxide), and
ii) at least one hydrophilic group comprising at least one cationic group selected from among:
- a linear or branched alkyl group having 1 to 12 carbon atoms and interrupted and/or substituted by at least one cationic group, and
- a polymeric hydrophilic group comprising at least one cationic group; and
c) 10 to 55 mole % of a co-surfactant comprising at least one polyethylene oxide) chain comprising at least 25 ethylene oxide units;
d) a solubilizing lipid comprising at least one fatty acid glyceride;
e) optionally a helper lipid;
f) a htiRNA hybrid according to claim 1 or 2,
wherein the molar percentages of amphiphilic lipid, of cationic surfactant and of co-surfactant are relative to the whole (amphiphilic lipid / cationic surfactant / co-surfactant / optional helper lipid).

8. Use of a htiRNA hybrid according to claim 1 or 2 to stabilize a complex between said antisense RNA and a nanoparticle.

9. A method for inserting in a eukaryote cell an antisense RNA able to modulate endogenous mechanisms of RNA interference, comprising placing the eukaryote cell in contact with the complex according to any of claims 3 to 7.

10. The complex according to any of claims 3 to 7 for use thereof in the prevention or treatment of a disease, in particular a disease of the digestive system such as chronic inflammatory disorders of the intestine, in particular Crohn's disease or haemorrhagic rectocolitis.
